# EUROPEAN PATENT SPECIFICATION

(11) **EP 4 395 384 B1**
(45) Date of publication and mention of the grant of the patent: **26.11.2025**
(21) Application number: 23829661.0
(22) Date of filing: 25.04.2023
(51) Int. Cl.: H04W 4/90, A61B 5/00, G08B 25/00, G08B 25/01, H04M 1/72418, H04W 4/38, H04W 48/18, A61B 5/021, A61B 5/024, A61B 5/11, A61B 5/145, A61B 5/1455, H04W 88/06, H04W 4/80, H04W 76/50

(54) **HELP-SEEKING INFORMATION SENDING METHOD AND SYSTEM**
VERFAHREN UND SYSTEM ZUM SENDEN VON HILFESUCHENDEN INFORMATIONEN
PROCÉDÉ ET SYSTÈME D'ENVOI D'INFORMATIONS DE RECHERCHE D'AIDE

(30) Priority: 30.06.2022 CN 202210759989
(43) Date of publication of application: 03.07.2024
(73) Proprietor: Honor Device Co., Ltd., Shenzhen, Guangdong 518040 (CN)
(72) Inventor: SUN, Xiaoyu, Shenzhen, Guangdong 518040 (CN); ZHAO, Chen, Shenzhen, Guangdong 518040 (CN); GONG, Weilin, Shenzhen, Guangdong 518040 (CN); ZHI, Gang, Shenzhen, Guangdong 518040 (CN)
(74) Representative: Isarpatent
(86) International application number: PCT/CN2023/090704
(87) International publication number: WO 2024/001474

(56) References cited:
- CN-A- 106 454 724
- CN-A- 110 058 277
- CN-A- 113 784 287
- CN-A- 113 784 287
- US-A1- 2009 224 966
- US-A1- 2014 148 118
- US-B1- 8 249 547

## Description

### TECHNICAL FIELD

This application relates to the field of terminal technologies, and in particular, to a distress information sending method and system, and a terminal device.

### BACKGROUND

When encountering disasters and danger (such as accidents during outdoor sports, natural disasters such as earthquakes and typhoons, ship damages during an ocean travel, or animals attacking during field scientific research), people can call for help from the outside world through mobile phones as soon as possible.

In the process of calling for help by using the mobile phones, most users call for help by using a cellular network of the mobile phones, for example, a user makes a distress call to the outside world by using the cellular network. When locations of the users are not covered by a cellular network, the users usually have to give up calling for help by using the mobile phones. As a result, the users are not to be rescued in time.

CN 106 454 724 A discloses a positioning rescue system and method, the system comprising: a user terminal for monitoring mobile network signals; and when monitoring fails to receive mobile network signals, also for initiating a channel to connect to a satellite device to send a rescue message; a satellite device for connecting the user terminal to a satellite receiving device, establishing network communication between the server and the user terminal according to the satellite network; and a server for switching between the mobile network and the satellite network, and establishing network communication with the user terminal.

CN 110 058 277 A discloses a distress request method and system based on satellite positioning and a mobile network. When a user needs to request assistance and sends a distress request, the method and system first obtain the current latitude and longitude information of the user, query the geographical location information corresponding to the current latitude and longitude information through the mobile network, and send the user's distress information and geographical location information to the rescuers. The method and system also display the current location of the rescuers and the user, the optimal route, and the time required on a map for the rescuers, so that the rescuers can conveniently arrive at the user's location in a timely manner and rescue efficiency can be improved.

US 2009 224966 A1 discloses an emergency communication device capable of transmitting an emergency alert signal using more than one wireless communication system.

### SUMMARY

In view of this, the application provides a distress information sending method and system, and a terminal device, as defined in the appended set of claims, to enable a user to be rescued in time by setting automatic switching of various communication means.

According to a first aspect, this application provides a sending distress information method, and the method includes: A terminal device obtains first distress information, where the first distress information includes at least one of adverse sign information or positioning information of a user. The terminal device selects a first communication network from a plurality of communication networks in a preset sequence, where the first communication network is any one of a short-range connection network, a cellular network, a satellite network, and a communication network between remote devices, and the communication network between remote devices is a point-to-point communication network established by the terminal device and a rescue processing device by using a preset frequency resource. When the terminal device can be connected to the rescue processing device by using the first communication network, the terminal device sends the first distress information to the rescue processing device by using the first communication network. Alternatively, when the terminal device cannot be connected to the rescue processing device by using the first communication network, the terminal device re-selects a new first communication network from the plurality of communication networks in the preset sequence.

In the solution of this application, the communication network can be automatically switched in the preset sequence, so that a network capable of sending the first distress information can be selected from the plurality of communication networks, so that the user can be rescued in time.

In a possible implementation, before that a terminal device obtains first distress information, the method further includes: The terminal device receives first pre-distress information sent from a wearable smart device, where the first pre-distress information is generated based on adverse sign information when the wearable smart device detects an abnormality in a sign of the user; and that a terminal device obtains first distress information includes: The terminal device generates the first distress information based on the first pre-distress information.

In the solution of this application, the adverse sign information of the user is detected by the wearable smart device, so that a current physical state of the user can be obtained. Therefore, accurate reference information can be provided for subsequent rescue.

In a possible implementation, that the terminal device generates the first distress information based on the first pre-distress information includes: The terminal device sorts distress content in the first pre-distress information based on an importance degree; and processes distress content corresponding to a sequence number before a preset threshold, to obtain the first distress information.

In the solution of this application, the distress content in the first pre-distress information is sorted based on an importance degree. This can facilitate a rescue team to quickly identify important information in the first pre-distress information after receiving the first pre-distress information. Generally, after receiving the first pre-distress information, it is necessary to perform information analysis on the first pre-distress information, but in this application, there is no need to perform information analysis on the first pre-distress information. This can greatly reduce time consumption caused by the information analysis, and therefore improves rescue efficiency.

The preset sequence from front to back is: the short-range connection network, the cellular network, the satellite network, and the communication network between remote devices.

Usually, use costs of a short-range connection module, a cellular communication module, and a satellite communication module increase in sequence, and a module for communication between remote devices requires that two devices turn on this function at the same time to implement communication. This is difficult to use. Therefore, a communication manner in which communication is performed by using the short-range connection module first, and when communication fails, communication is performed by using the cellular communication module, the satellite communication module, and the module for communication between remote devices in sequence. This can reduce use costs, and ensure smooth sending of distress information as much as possible, so that a distressed person can be rescued in time.

In a possible implementation, that the terminal device selects a first communication network from a plurality of communication networks in a preset sequence, and when the terminal device can be connected to the rescue processing device by using the first communication network, the terminal device sends the first distress information to the rescue processing device by using the first communication network; or when the terminal device cannot be connected to the rescue processing device by using the first communication network, the terminal device re-selects a new first communication network from the plurality of communication networks in the preset sequence includes: The terminal device selects the short-range connection network as the first communication network from the plurality of communication networks in the preset sequence, and determines whether the terminal device can be connected to the rescue processing device by using the short-range connection network. When the terminal device can be connected to the rescue processing device by using the short-range connection network, the terminal device sends the first distress information to the rescue processing device by using the short-range connection network. Alternatively, when the terminal device cannot be connected to the rescue processing device by using the short-range connection network, the terminal device selects the cellular network as the first communication network, and determines whether the terminal device can be connected to the rescue processing device by using the cellular network. When the terminal device can be connected to the rescue processing device by using the cellular network, the terminal device sends the first distress information to the rescue processing device by using the cellular network. Alternatively, when the terminal device cannot be connected to the rescue processing device by using the cellular network, the terminal device selects the satellite network as the first communication network, and determines whether the terminal device can be connected to the rescue processing device by using the satellite network. When the terminal device can be connected to the rescue processing device by using the satellite network, the terminal device sends the first distress information to the rescue processing device by using the satellite network. Alternatively, when the terminal device cannot be connected to the rescue processing device by using the satellite network, the terminal device selects the communication network between remote devices as the first communication network, and determines whether the terminal device can be connected to the rescue processing device by using the communication network between remote devices. When the terminal device can be connected to the rescue processing device by using the communication network between remote devices, the terminal device sends the first distress information to the rescue processing device by using the communication network between remote devices. Alternatively, when the terminal device cannot be connected to the rescue processing device by using the communication network between remote devices, the terminal device re-selects a new first communication network in the preset sequence.

In a possible implementation, the determining whether the terminal device can be connected to the rescue processing device by using the communication network between remote devices includes: The terminal device initiates scanning of a communication signal between the remote devices, and when the communication signal between the remote devices is obtained through scanning, the terminal device establishes a connection with a peer device that sends the communication signal between the remote devices. The terminal device sends an inquiry instruction to the peer device, where the inquiry instruction is used for inquiring whether the peer device can be connected to the rescue processing device, and the inquiry instruction includes an address of the rescue processing device. In response to an inquiry result sent by the peer device, when the peer device can be connected to the rescue processing device, the terminal device determines that the terminal device can be connected to the rescue processing device by using the communication network between remote devices, or when the peer device cannot be connected to the rescue processing device, the terminal device determines that the terminal device cannot be connected to the rescue processing device by using the communication network between remote devices.

In a possible implementation, the determining whether the terminal device can be connected to the rescue processing device by using the cellular network includes: The terminal device determines whether the terminal device can be connected to the rescue processing device by using a subscriber identity identification SIM card operator cellular network in the cellular network. When the terminal device can be connected to the rescue processing device by using the SIM card operator cellular network in the cellular network, the terminal device determines that the first communication network is the SIM card operator cellular network in the cellular network. Alternatively, when the terminal device cannot be connected to the rescue processing device by using the SIM card operator cellular network in the cellular network, the terminal device determines whether the terminal device can be connected to the rescue processing device by using a non-SIM card operator cellular network in the cellular network. When the terminal device can be connected to the rescue processing device by using a non-SIM card operator cellular network in the cellular network, the terminal device determines that the first communication network is the non-SIM card operator cellular network in the cellular network. Alternatively, the method further includes: When the terminal device cannot be connected to the rescue processing device by using a non-SIM card operator cellular network in the cellular network, the terminal device determines whether the terminal device can be connected to the rescue processing device by using the satellite network.

In a possible implementation, the method further includes: The terminal device receives second pre-distress information sent from the wearable smart device, where the second pre-distress information is generated based on changed sign information when the wearable smart device detects that sign information of the user is inconsistent with the adverse sign information in the first pre-distress information. The terminal device generates second distress information based on the second pre-distress information and sends the second distress information to the rescue processing device.

In a possible implementation, the method further includes: The terminal device receives third pre-distress information sent from the wearable smart device, where the third pre-distress information is generated based on changed positioning information when the wearable smart device detects that positioning information of the user is inconsistent with the positioning information in the first pre-distress information. The terminal device generates third distress information based on the third pre-distress information and sends the third distress information to the rescue processing device. Alternatively, when the terminal device detects that positioning information of the user is inconsistent with the positioning information in the first distress information, the terminal device collects the changed positioning information, generates fourth distress information based on the changed positioning information, and sends the fourth distress information to the rescue processing device.

In a possible implementation, the method further includes: The terminal device receives rescue information sent from the rescue processing device. The rescue information is used to indicate the terminal device to establish contact with a rescue team.

In a possible implementation, the rescue information includes rescue time and a communication mode between the rescue team and the user. After the terminal device receives the rescue information sent from the rescue processing device, the method further includes: The terminal device establishes, based on the communication mode between the rescue team and the user, a connection with the rescue team when the rescue time arrives.

In the solution of this application, the rescue time in the rescue information can be used to remind the user to make preparations in advance before arrival of the rescue time, to facilitate timely rescue.

In a possible implementation, the first pre-distress information further includes at least one of user personal information, positioning information, emergency information, and situated environment information. The adverse sign information includes at least one of a blood type, blood pressure, blood oxygen content, and a heart rate. The personal information includes at least one of a gender and an age. The emergency information includes at least one of a home address, an allergic reaction, an emergency contact name, and an emergency contact phone number. The situated environment information includes at least one of a photo of a situated environment and a video of the environment.

In the solution of this application, the first pre-distress information includes the user personal information, the positioning information, the emergency information, situated environment information, and the like. The rescue team can provide more accurate rescue for a user by obtaining more user information.

In a possible implementation, the sending the second distress information to the rescue processing device includes: The terminal device determines a second communication network in the preset sequence, where the second communication network is used for establishing a connection with the rescue processing device, and the second communication network is any one of the short-range connection network, the cellular network, the satellite network, and the communication network between remote devices.

In a possible implementation, the short-range connection network includes at least one of a wireless fidelity Wi-Fi network, a Bluetooth network, and a ZigBee ZigBee protocol network.

According to a second aspect, a distress information sending system is provided, the distress information sending system includes a wearable smart device, a terminal device, and a rescue processing device. The wearable smart device, the terminal device, and the rescue processing device are configured to perform the distress information sending method according to any one of the first aspect.

According to a third aspect, an electronic device is provided. The electronic device has a function of performing the method according to the foregoing first aspect. The function may be implemented by using hardware, or may be implemented by hardware executing corresponding software. The hardware or software includes one or more modules corresponding to the above function.

According to a fourth aspect, an electronic device is provided, including: a processor and a memory. The memory is configured to store computer execution instructions, and when the electronic device is running, the processor executes the computer execution instructions stored in the memory to enable the electronic device to execute the distress information sending method according to any one of the first aspect.

For technical effects brought by any one of the design manners in the third aspect to the seventh aspect, refer to technical effects brought by different design manners in the first aspect. Details are not described herein again.

### BRIEF DESCRIPTION OF DRAWINGS

FIG. 1 is a schematic diagram 1 of a rescue scenario according to an embodiment of this application;
FIG. 2 is a schematic diagram of a structure of a distress information sending system according to an embodiment of this application;
FIG. 3 is a schematic diagram of a hardware structure of a second electronic device according to an embodiment of this application;
FIG. 4 is a schematic diagram of a software structure of a second electronic device according to an embodiment of this application;
FIG. 5A to FIG. 5C are a schematic diagram 1 of displays according to an embodiment of this application;
FIG. 6 is a schematic diagram 2 of displays according to an embodiment of this application;
FIG. 7A and FIG. 7B are a schematic flowchart 1 of a distress information sending method according to an embodiment of this application;
FIG. 8 is a schematic diagram 3 of displays according to an embodiment of this application;
FIG. 9A and FIG. 9B are a schematic flowchart 2 of a distress information sending method according to an embodiment of this application;
FIG. 10A to FIG. 10C are a schematic flowchart 3 of a distress information sending method according to an embodiment of this application;
FIG. 11A to FIG. 11C are a schematic flowchart 4 of a distress information sending method according to an embodiment of this application;
FIG. 12A to FIG. 12C are a schematic flowchart 5 of a distress information sending method according to an embodiment of this application;
FIG. 13 A to FIG. 13C are a schematic flowchart 6 of a distress information sending method according to an embodiment of this application;
FIG. 14 is a schematic diagram 2 of a rescue scenario according to an embodiment of this application;
FIG. 15 is a schematic diagram 3 of a rescue scenario according to an embodiment of this application;
FIG. 16 is a schematic diagram 4 of a rescue scenario according to an embodiment of this application; and
FIG. 17 is a schematic diagram of a structure of a chip system according to an embodiment of this application.

### DESCRIPTION OF EMBODIMENTS

Technical solutions in embodiments of this application are described below with reference to the accompanying drawings in the embodiments of this application. In the descriptions of this application, unless otherwise specified, "at least one" means one or more, and "a plurality of" means two or more. In addition, to clearly describe the technical solutions in embodiments of this application, in embodiments of this application, words such as "first" and "second" are used for distinguishing between same items or similar items that have basically same functions or purposes. A person skilled in the art may understand that the words such as "first" and "second" do not limit a quantity or an execution sequence, and the words such as "first" and "second" do not indicate a definite difference.

With development of electronic and communication technologies, portable terminals have become necessities of modern life, and with development of terminal technologies, functions of the portable terminals are more diversified. For example, a portable communication terminal can be used as a personal digital assistant in addition to being able to perform communication. Moreover, a portable terminal can also be configured in a multimedia center, to provide a user with all-round leisure services from listening to music, receiving multimedia digital broadcasts, watching movies to running various games. In addition, the portable terminal also integrates a function of a digital camera, and can take a picture and record a video.

Therefore, people are increasingly dependent on portable terminals. When users encounter natural disasters or man-made disasters, people usually choose to call for help to the outside world as soon as possible by using the portable terminal that is an electronic device carried at all times. The natural disasters are abnormal phenomena in nature that human beings depend on, mainly including earthquakes, volcanic eruptions, mudslides, tsunamis, typhoons, floods, and the like. The man-made disasters are disasters caused by human factors, mainly including natural resources exhaustion disasters, environmental pollution disasters, fires, traffic accidents, and the like. Whether it is a natural disaster or man-made disaster, as long as the disaster occurs, the disaster may cause serious consequences.

At present, distress can be spread through various communication means, for example, short-range communication, cellular communication, satellite communication, and communication between remote devices. However, these communication means are relatively independent, and only when a user starts a specific communication means is the corresponding communication means started. For a crisis moment of emergency rescue, the user cannot know what kind of communication means can be used to immediately send out distress information, and can only try one by one. Moreover, this distress mode needs great cooperation from the user. If a situation faced by users is bad, and it is inconvenient for the user to operate or the user has no condition to perform an operation, this distress mode provide no help for the user, and therefore the user miss a best rescue opportunity.

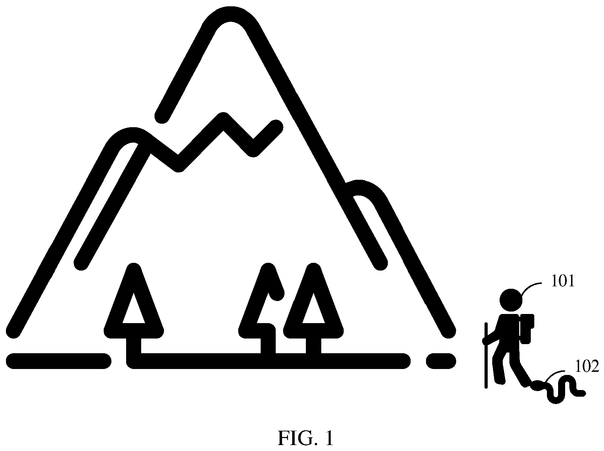

For example, FIG. 1 illustrates a possible distress scenario in which a user 101 conducts a field research expedition in a deep mountain, and in a process of research expedition, accidentally steps on a poisonous snake 102, and was attacked by the poisonous snake 102. Some time after being bitten by the poisonous snake, a smart watch of the user 101 detects that blood pressure of the user 101 begins to decrease, and notifies the user 101 in a form of lighting. After seeing the lighting prompt of the smart watch, the user 101 knows that a sign of the user is abnormal. By looking at the smart watch, it can be seen that the blood pressure drops beyond a normal value. This is life-threatening. Then the user 101 makes a distress call to the outside world by using a mobile phone. Because of weak signal coverage of a base station in deep mountains, the distress call of the user 101 cannot be dialed out. When the user 101 does not find another viable mean of communication, the user may not be able to contact the outside world and thus be unable to be rescued in time.

Therefore, this application provides distress information sending methods and a distress information sending system. By setting automatic switching of various communication means, a user can be rescued in time.

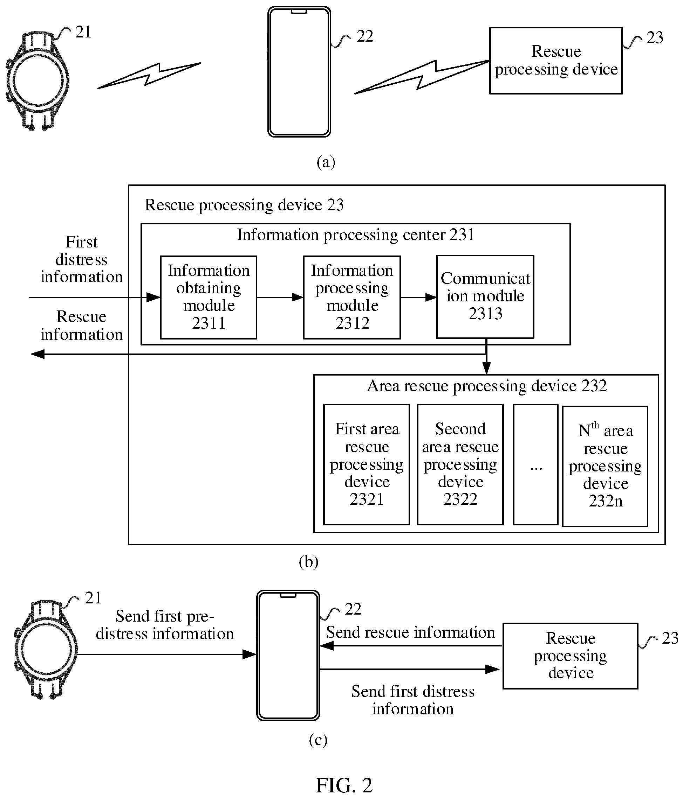

The distress information sending methods provided in embodiments of this application can be applied to the distress information sending system. In (a) in FIG. 2, a distress information transmission system 20 is shown, and the distress information transmission system 20 includes a first electronic device 21, a second electronic device 22, and a rescue processing device 23. The first electronic device 21 and the second electronic device 22 may be connected by any possible connection mode like a wired network or a wireless network, and the second electronic device is connected to the rescue processing device by using a wireless network.

Specifically, the first electronic device 21 is configured to collect adverse sign information of a user and send the collected adverse sign information of the user to the second electronic device.

The first electronic device 21 may be a wearable smart device. The wearable smart device can be applied in outdoor sports and fitness fields and medical care fields. In the outdoor sports and fitness fields, the wearable smart device is represented by a lightweight watch, a bracelet, an accessory, and the like, and is mainly configured to monitor and analyze sports data. For example, sports data may include a heart rate, a step frequency, atmospheric pressure, a diving depth, an altitude, and the like.

In medical care fields, the wearable smart device is represented by a medical vest, a belt, an implantable chip, and the like, and is mainly configured to monitor and analyze sign data. For example, sign data may include blood pressure, a heart rate, and the like.

At present, common wearable smart devices can be smart watches, smart bracelets, smart glasses, smart headbands, drum T-shirts, social jeans, satellite navigation pants, wearable multi-touch projectors, and the like. For example, in (a) in FIG. 2, the first electronic device 21 is illustrated by using a smart watch as an example.

The second electronic device 22 is configured to: receive first pre-distress information including the adverse sign information of the user sent from the first electronic device 21, process the received first pre-distress information to generate the first distress information, and finally send the first distress information to the rescue processing device 23. The second electronic device 22 may also receive rescue information from the rescue processing device 23 and display the rescue information to the user. The second electronic device 22 may be a mobile phone, a tablet computer, a desktop computer, a handheld computer, a notebook computer, a vehicle-mounted device, an ultra-mobile personal computer (ultra-mobile personal computer, UMPC), a netbook, a personal digital assistant (PDA), an augmented reality device, a virtual reality device, and the like. A specific form of the second electronic device is not particularly limited in embodiments of this application. In (a) in FIG. 2, the second electronic device 22 is illustrated by using a mobile phone as an example. The distress information sending methods provided in embodiments of the application can be applied to the second electronic device.

The rescue processing apparatus 23 is configured to: receive the first distress information sent from the second electronic apparatus 22, generate the rescue information based on the first distress information, and then assign a nearest rescue team to the distressed person based on the rescue information. As shown in (b) in FIG. 2, the rescue processing device 23 includes an information processing center 231 and an area rescue processing device 232. The information processing center 231 may specifically include a plurality of modules. For example, the information processing center 231 includes an information obtaining module 2311, an information processing module 2312, and a communication module 2313. The area rescue processing device 232 may also specifically include a plurality of modules and, for example, the area rescue processing device 232 includes a first area rescue processing device 2321, a second area rescue processing device 2322, ..., and an N^{th} area rescue processing device 232n.

The information processing center 231 is configured to: collect the first distress information, and generate the rescue information based on the first distress information. The information processing center 231 may be implemented by the following modules, and specifically includes:
the information obtaining module 2311, configured to collect first distress information sent by the distressed person by using a communication device;
the information processing module 2312, configured to: analyze and process the obtained first distress information, obtain the rescue information, and send the rescue information to the distressed person and the area rescue processing device 232 through the communication module 2313; and
the communication module 2313, configured to: send the rescue information generated by the information processing module to the distressed person and the area rescue processing device 232.

The area rescue processing device 232 is configured to: receive the rescue information and assign the nearest rescue team for the distressed person based on a to-be-rescued area in the rescue information. The area rescue processing device 232 may be implemented by the following modules, and specifically includes:
the first area rescue processing device 2321, configured to: receive the rescue information sent from the area rescue processing device, and perform a rescue operation for a first area in the rescue information;
the second area rescue processing device 2322, configured to: receive the rescue information sent from the area rescue processing device, and perform a rescue operation for a second area in the rescue information; and
the N^{th} area rescue processing device 232n, configured to: receive the rescue information sent from the area rescue processing device, and perform a rescue operation for an N^{th} area in the rescue information.

The following uses the smart watch and the mobile phone as an example, interaction between devices involved in the distress information sending methods provided in embodiments of this application are explained.

IWith reference to (c) in FIG. 1 and FIG. 2, after the user 101 is bitten by the poisonous snake, the smart watch (for example, the first electronic device) can detect that sign information of the user 101 (for example, a blood pressure drop of the user 101) changes, and after determining that a change amplitude of the sign information exceeds a preset threshold, the smart watch sends the detected first pre-distress information including the adverse sign information of the user to the mobile phone (the second electronic device). After receiving the first pre-distress information sent by the smart watch, the mobile phone generates the first distress information based on the first pre-distress information, and sends the first distress information to the rescue processing device by using the communication module in the mobile phone. After receiving the first distress information sent by the mobile phone, the rescue processing device generates the rescue information based on the first distress information and sends the rescue information to the mobile phone. After receiving the rescue information, the mobile phone displays the rescue information to the user 101 by using a display of the mobile phone. After seeing the rescue information displayed by the mobile phone, the user 101 can wait for rescue based on the rescue information. The communication module of the mobile phone can be a short-range network, a cellular network, a satellite communication network, or a communication network between remote devices, to communicate with the rescue processing device.

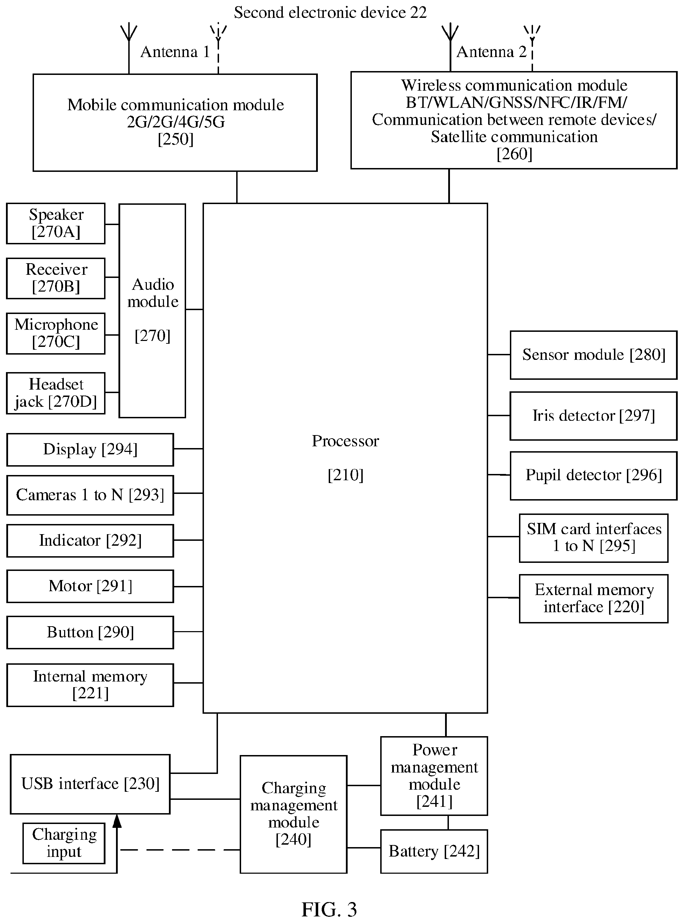

FIG. 3 is a schematic diagram of a hardware structure of the second electronic device 22 according to an embodiment of this application. The electronic device 22 may include a processor 210, an external memory interface 220, an internal memory 221, a universal serial bus (universal serial bus, USB) interface 230, a charging management module 240, a power management unit 241, a battery 242, an antenna 1, an antenna 2, a mobile communication module 250, a wireless communication module 260, an audio module 270, a loudspeaker 270A, a phone receiver 270B, a microphone 270C, a headset jack 270D, a sensor module 280, a key 290, a motor 291, an indicator 292, a camera 293, a display 294, a subscriber identification module (subscriber identification module, SIM) card interface 295, a pupil detector 296, an iris detector 297, and the like.

The sensor module 280 may include a pressure sensor, a gyro sensor, a barometric pressure sensor, a magnetic sensor, an acceleration sensor, a distance sensor, an optical proximity sensor, a fingerprint sensor, a temperature sensor, and a touch sensor, an ambient light sensor, a bone conduction sensor, and the like.

It may be understood that the structure shown in this embodiment does not constitute a specific limitation on the second electronic device 22. In some other embodiments, the second electronic device 22 may include more or fewer components than those shown in the figure, or some components may be combined, or some components may be split, or a different component arrangement may be used. The components shown in the figure may be implemented in hardware, software, or a combination of software and hardware.

The processor 210 may include one or more processing units. For example, the processor 210 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, and/or a neural-network processing unit (neural-network processing unit, NPU). Different processing units may be separate components, or may be integrated into one or more processors.

The controller may be a nerve center and a command center of the second electronic device 22. The controller may generate an operation control signal based on instruction operation code and a timing signal, to implement control on instruction fetching and execution.

A memory may be further disposed in the processor 210, and is configured to store instructions and data. In some embodiments, the memory in the processor 210 is a cache. The memory may store instructions or data recently used or cyclically used by the processor 210. When the processor 210 needs to use the instructions or the data again, the processor may directly invoke the instructions or the data from the memory. This avoids repeated access, reduces waiting time of the processor 210, and improves system efficiency.

In some embodiments, the processor 210 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

It may be understood that an interface connection relationship between the modules shown in this embodiment is merely an example for description, and constitutes no limitation on the structure of the second electronic device 22. In some other embodiments, the second electronic device 22 may alternatively use an interface connection manner different from that in the foregoing embodiment, or use a combination of a plurality of interface connection manners.

The charge management module 240 is configured to receive a charging input from the charger. When charging the battery 242, the charging management module 240 may further supply power to the second electronic device 22 by using the power management module 241.

The power management module 241 is configured to be connected to the battery 242, the charging management module 240, and the processor 210. The power management module 241 receives input from the battery 242 and/or the charging management module 240, and supplies power to the processor 210, the internal memory 221, the external memory, the display 294, the camera 293, the wireless communication module 260, and the like. In some other embodiments, the power management module 241 may alternatively be provided in the processor 210. In some other embodiments, the power management module 241 and the charging management module 240 may alternatively be disposed in a same component.

A wireless communication function of the second electronic device 22 may be implemented by using the antenna 1, the antenna 2, the mobile communication module 250, the wireless communication module 260, the modem processor, the baseband processor, and the like.

The antenna 1 and the antenna 2 are configured to transmit and receive electromagnetic wave signals. Each antenna of the electronic device 22 may be configured to cover one or more communication frequency bands. Different antennas may further be reused to improve utilization of the antennas. For example, the antenna 1 may be reused as a diversity antenna of a wireless local area network.

The mobile communication module 250 may provide a solution to wireless communication such as 2G/3G/4G/5G applied to the second electronic device 22. The mobile communication module 250 may include at least one filter, a switch, a power amplifier, a low noise amplifier (low noise amplifier, LNA), and the like. The mobile communication module 250 may receive an electromagnetic wave by using the antenna 1, perform processing such as filtering or amplification on the received electromagnetic wave, and transmit the electromagnetic wave to the modem processor for demodulation. The mobile communication module 250 may further amplify a signal modulated by the modem processor, and convert the signal into an electromagnetic wave by using the antenna 1 for radiation.

The modem processor may include a modulator and a demodulator. The modulator is configured to modulate to-be-sent low frequency baseband signals into medium and high frequency signals. The demodulator is configured to demodulate a received electromagnetic wave signal into a low frequency baseband signal. Then the demodulator transmits the demodulated low frequency baseband signal to the baseband processor for processing. The low-frequency baseband signal is processed by the baseband processor and then transferred to an application processor. The application processor outputs a sound signal by using an audio device (which is not limited to the speaker 270A, the receiver 270B, or the like), or displays an image or a video by using the display 294.

The wireless communication module 260 may provide a solution for wireless communication solution including a wireless local area network (wireless local area network, WLAN) (for example, a wireless fidelity (wireless fidelity, Wi-Fi) network), Bluetooth (Bluetooth, BT), and a global navigation satellite system (global navigation satellite system, GNSS), frequency modulation (frequency modulation, FM), a near field communication (near field communication, NFC) technology, an infrared (infrared, IR) technology, communication between remote devices, satellite communication, and the like to be applied to the second electronic device 22. The wireless communication module 260 may be one or more components into which at least one communication processing module is integrated. The wireless communication module 260 receives an electromagnetic wave through the antenna 2, performs frequency modulation and filtering processing on the electromagnetic wave signal, and sends the processed signal to the processor 210. The wireless communication module 260 may further receive a to-be-sent signal from the processor 210, perform frequency modulation and amplification on the signal, and convert the signal into an electromagnetic wave for radiation through the antenna 2.

In some embodiments, the antenna 1 and the mobile communication module 250 in the second electronic device 22 are coupled, and the antenna 2 and the wireless communication module 260 in the second electronic device 22 are coupled, so that the second electronic device 22 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), the BT, the GNSS, the WLAN, the NFC, the FM, the IR technology, and/or the like. The GNSS may include a global positioning system (global positioning system, GPS), a global navigation satellite system (global navigation satellite system, GLONASS), a BeiDou navigation satellite system (beidou navigation satellite system, BDS), a quasi-zenith satellite system (quasi-zenith satellite system, QZSS), and/or a satellite based augmentation system (satellite based augmentation systems, SBAS).

The second electronic device 22 implements a display function by using the GPU, the display 294, the application processor, and the like. The GPU is a microprocessor for image processing and connects the display 294 and the application processor. The GPU is configured to: perform mathematical and geometric computation, and render an image. The processor 210 may include one or more GPUs that execute program instructions to generate or change display information.

The display 294 is configured to display an image, a video, and the like. The display 294 includes a display panel. The display panel may be a liquid crystal display (liquid crystal display, LCD), a light-emitting diode (light-emitting diode, LED) an organic light-emitting diode (organic light-emitting diode, OLED), an active-matrix organic light emitting diode (active-matrix organic light emitting diode, AMOLED), a flexible light-emitting diode (flex light-emitting diode, FLED), a mini-LED, a micro-LED, a micro-LED, a quantum dot light emitting diode (quantum dot light emitting diode, QLED), and the like.

In this embodiment of this application, when the display 294 is integrated with a touch sensor, the display 294 may be referred to as a touchscreen. The touch sensor may be also referred to as a "touch panel". That is, the display 294 may include a display panel and a touch panel. The touch sensor is configured to detect a touch operation performed on or near the touch sensor. After the touch sensor detects the touch operation, the touch sensor can trigger a driver of a kernel layer (for example, a TP driver) to periodically scan a touch parameter generated by the touch operation. Then, the driver of the kernel layer transfers the touch parameter to a related module in an upper layer for the related module to determine a touch event type corresponding to the touch parameter.

In addition, the display 294 may provide a visual output related to the touch operation. In some other embodiments, the touch sensor may alternatively be disposed on a surface of the second electronic device 22 rather than integrated in the display 294. In this case, the touch sensor is located on a location different from that of the display 294. In embodiments of this application, that a screen is a screen integrated with a touch sensor is used as an example, and a specific process of a display method of playing interfaces is explained.

The second electronic device 22 may implement a photographing function by using the ISP, the camera 293, the video codec, the GPU, the display 294, the application processor, and the like. The ISP is configured to process data fed back by the camera 293. The camera 293 is configured to capture a still image or a video. The digital signal processor is configured to process a digital signal, and may process another digital signal in addition to the digital image signal. The video codec is configured to compress or decompress a digital video. The second electronic device 22 may support one or more video codecs. In this way, the second electronic device 22 may play or record videos in a plurality of encoding formats, for example, moving picture experts group (moving picture experts group, MPEG)-1, MPEG-2, MPEG-3, and MPEG-4.

The NPU is a neural-network (neural-network, NN) computing processor, and quickly processes input information by emulating a biological neural network structure, for example, by emulating a mode of transfer between human-brain neurons, and may further perform self-learning constantly. Applications such as intelligent cognition of the second electronic device 22 may be implemented through the NPU, for example, image recognition, facial recognition, speech recognition, and text understanding.

The external memory interface 220 may be configured to be connected to an external storage card, for example, a micro SD card, to expand a storage capability of the second electronic device 22. The external memory card communicates with the processor 210 through the external memory interface 220, to implement a data storage function. For example, files such as music and videos are stored in the external storage card. The internal memory 221 may be configured to store computer-executable program code. The executable program code includes instructions. The processor 210 runs the instructions stored in the internal memory 221, to perform various function applications and data processing of the second electronic device 22. For example, in this embodiment of this application, the processor 210 may execute the instructions stored in the internal memory 221. The internal memory 221 may include a program storage area and a data storage area. The program storage area may store an operating system, an application required for at least one function (like a sound playing function and an image display function), and the like. The data storage area may store data (like audio data and a phone book) created based on use of the second electronic device 22, and the like. In addition, the internal memory 221 may include a high-speed random access memory, or may include a nonvolatile memory, for example, at least one magnetic disk storage device, a flash memory, or a universal flash storage (universal flash storage, UFS).

The second electronic device 22 may implement an audio function like music play and recording through the audio module 270, the speaker 270A, the receiver 270B, the microphone 270C, the headset jack 270D, the application processor, and the like.

The audio module 270 is configured to convert digital audio information into an analog audio signal for output, and is also configured to convert analog audio input into a digital audio signal. The audio module 270 may be further configured to code and decode an audio signal. The speaker 270A, also referred to as a "horn", is configured to convert an audio electrical signal into a sound signal. The receiver 270B, also referred to as an "earpiece", is configured to convert an electrical audio signal into a sound signal. The microphone 270C, also referred to as a "mike" or a "mic", is configured to convert a sound signal into an electrical signal. The headset jack 270D is configured to connect to a wired headset.

The button 290 includes a power button, a volume button, and the like. The button 290 may be a mechanical button, or may be a touch button. The second electronic device 22 may receive a button input, and generate a button signal input related to user setting and function control of the second electronic device 22. The motor 291 may generate a vibration prompt. The motor 291 may be configured to provide an incoming call vibration prompt and touch vibration feedback. The indicator 292 may be an indicator light, and may be configured to indicate a charging status and a power change, or may be configured to indicate a message, a missed call, a notification, and the like. The SIM card interface 295 is configured to connect to a SIM card. The SIM card may be inserted into the SIM card interface 295 or removed from the SIM card interface 295, to implement contact with or separation from the second electronic device 22. The second electronic device 22 may support two or N SIM card interfaces, where N is a positive integer greater than 2. The SIM card interface 295 may support a nano-SIM card, a micro-SIM card, a SIM card, and the like.

A software system of the mobile phone may use a layered architecture, an event driven architecture, a micro core architecture, a micro service architecture, or a cloud architecture. In this embodiment of the present invention, an Android^{®} system of the hierarchical architecture is used as an example to describe the software structure of the mobile phone.

Software is divided into several layers by using the layered architecture, and each layer has a clear role and task. The layers communicate with each other through interfaces. In some embodiments, the Android^{®} system is divided into four layers: an application layer, an application framework layer, an Android runtime and system library, and a kernel layer from top down.

The application layer may include a series of application packages.

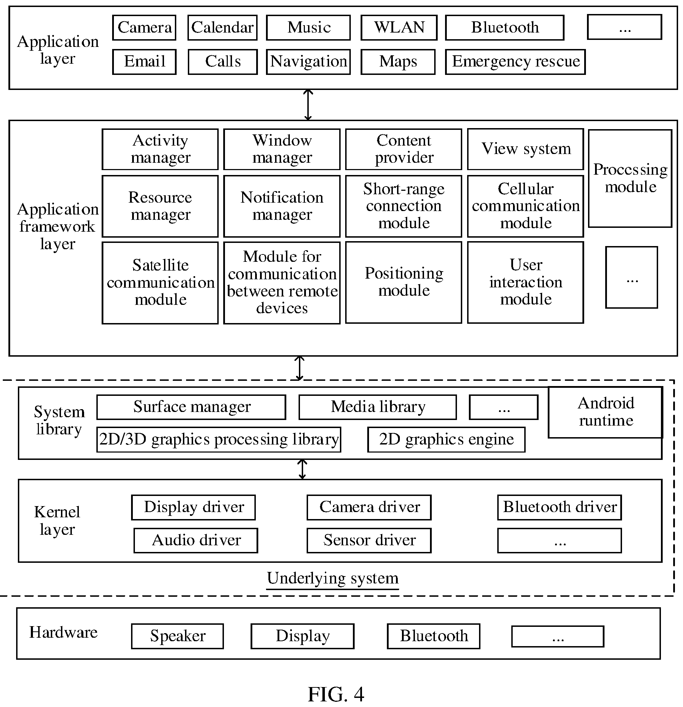

As shown in FIG. 4, the application package can include applications such as Camera, Mail, Calendar, Call, Music, Navigation, WLAN, Maps, Bluetooth, and Emergency rescue.

The application framework layer provides an application programming interface (application programming interface, API) and a programming framework for an application at the application layer. The application framework layer includes some predefined functions. For example, the application framework layer may include an activity manager, a window manager, a content provider, a view system, a resource manager, a notification manager, a short-range connection module, a cellular communication module, a satellite communication module, a module for communication between remote devices, a processing module, a user interaction module, and a positioning module, and the like. Embodiments of this application are not limited thereto.

The activity manager (Activity Manager) is configured to manage a lifecycle of each application. The application usually runs in an operating system in a form of an activity. For each activity, there is a corresponding activity record (ActivityRecord) in the activity manager, the activity manager records a status of an activity of the application. The activity manager can use this ActivityRecord as an identifier to schedule an activity process of the application.

The window manager (WindowManagerService) is configured to manage graphical user interface (GUI) resources used on the screen, and is specifically configured to: obtain a screen size, create and destruct a window, display and hide a window, lay out a window, manage a focus, manage an input method and a wallpaper, and the like.

The short-range connection module can perform information exchange with the rescue processing device according to a short-range connection communication protocol. For example, the short-range connection module sends the first distress information generated by the processing module to the rescue processing device. The short-range connection module can further perform information exchange with the wearable smart device, for example, obtain the first pre-distress information collected by the wearable smart device including the adverse sign information of the user.

The cellular communication module can perform information exchange with the rescue processing device according to a cellular communication protocol. For example, the cellular communication module sends the first distress information generated by the processing module to the rescue processing device.

The satellite communication module can perform information exchange with the rescue processing device according to a satellite communication protocol. For example, the satellite communication module sends the first distress information generated by the processing module to the rescue processing device.

The module for communication between remote devices may establish a temporary point-to-point communication link within a certain distance based on a specific frequency resource, and perform information exchange with the rescue processing device. For example, module for communication between remote devices sends the first distress information generated by the processing module of the mobile phone to the rescue processing device.

The processing module can receive the first pre-distress information sent from the short-range connection module and the first pre-distress information sent from the user interaction module, and generate the first distress information based on the first pre-distress information. Then, first distress information is sent to the rescue processing device by using any one of the short-range connection module, the cellular communication module, the satellite communication module, or the module for communication between remote devices.

The user interaction module is configured to: receive the first pre-distress information input from the user, and then send the first pre-distress information to the processing module. Specifically, the user may edit the first pre-distress information by performing a press operation on the second electronic device 22.

The positioning module obtains current location information of the user and sends the obtained location information to the processing module.

The system library, the kernel layer, and the like below the application framework layer may be referred to as an underlying system. The underlying system includes the underlying display system configured to provide a display service. For example, the underlying display system includes a display driver at the kernel layer and a surface manager in the system library.

The Android runtime (Android Runtime) includes a kernel library and a virtual machine. The Android runtime is responsible for scheduling and management of the Android system. The core library includes two parts: one part is a performance function that the Java language needs to invoke, and the other part is a core library of Android. The application layer and the application framework layer run on a virtual machine. The virtual machine executes Java files of the application layer and the application framework layer as binary files. The virtual machine is used to execute functions such as object lifecycle management, stack management, thread management, security and exception management, and garbage collection.

The system library may include a plurality of functional modules, For example, for example, a surface manager (surface manager), media libraries (media libraries), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media libraries support multiple common audio and video formats for playback and recording, as well as static image files. The media library may support a plurality of audio and video encoding formats, such as MPEG-4, H.264, MP3, AAC, AMR, JPG, and PNG.

OpenGL ES is configured to implement three-dimensional graphics drawing, image rendering, compositing, layer processing, and the like.

SGL is a drawing engine for 2D drawing.

The kernel layer is a layer between hardware and software. The kernel layer includes at least a display driver, a camera driver, an audio driver, and a sensor driver.

In some embodiments, when the user wishes to start a rescue function and can be rescued in time, personal information can be input in the first electronic device or the second electronic device in advance.

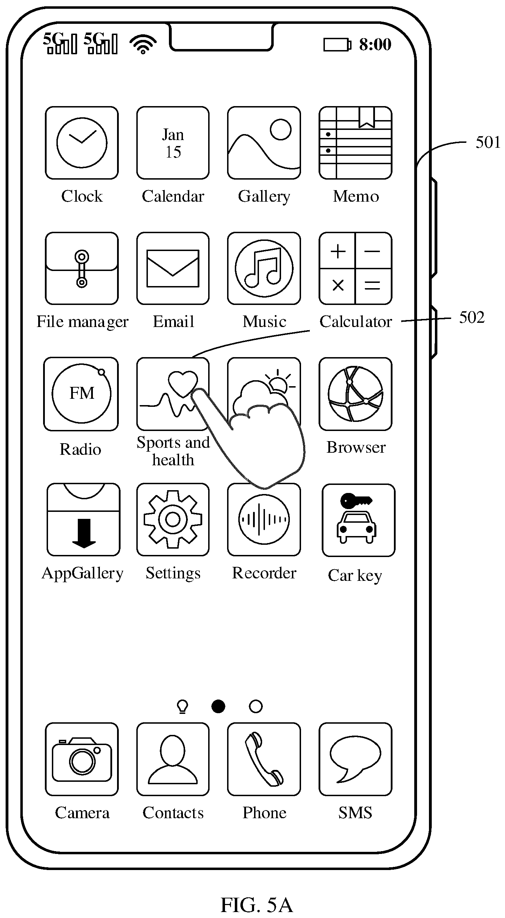

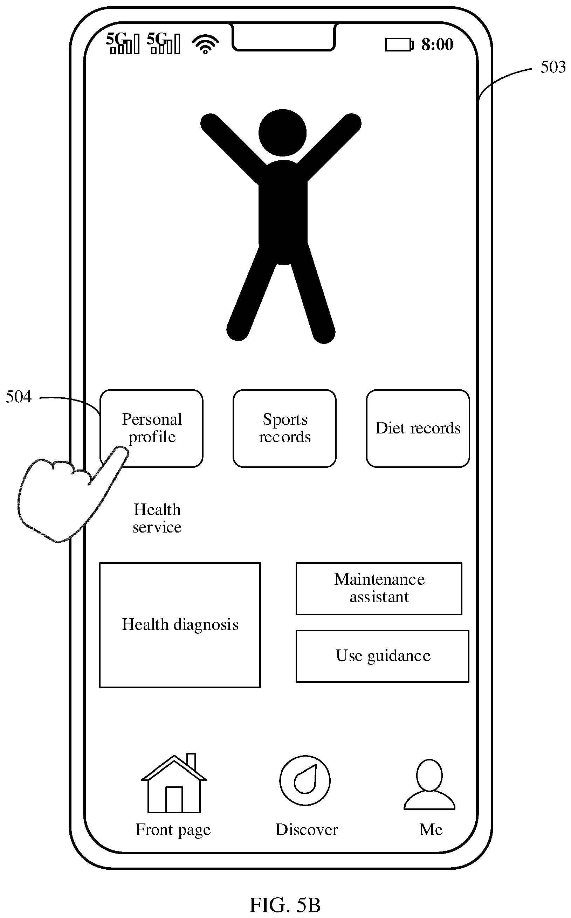

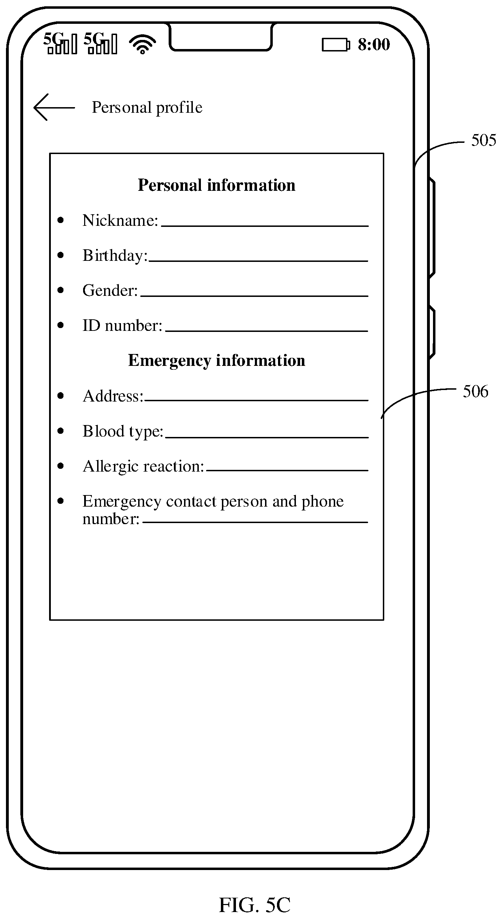

For example, that the second electronic device is a mobile phone is used as an example. As shown in FIG. 5A, the mobile phone can receive a tap operation of a user on an icon 502 of a Sports and health APP in a home screen interface (namely, a desktop) 501 of the mobile phone, and when the Sports and health APP is not running, the mobile phone can start the Sports and health APP in response to the tap operation. An interface 503 shown in FIG. 5B is also displayed. The interface 503 may include a personal profile function button 504. In response to an operation of the user on the personal profile function button 504 on the interface 503, the mobile phone may display an interface 505 as shown in FIG. 5C. The interface 505 may include a personal profile interface 506. The personal profile interface 506 includes personal information and emergency information, the personal information includes a nickname, a birthday, a gender, and an ID number, and the emergency information includes an address, a blood type, an allergic reaction, an emergency contact, and a phone number. The user can refine the personal information and the emergency information of the user in the personal profile interface 505.

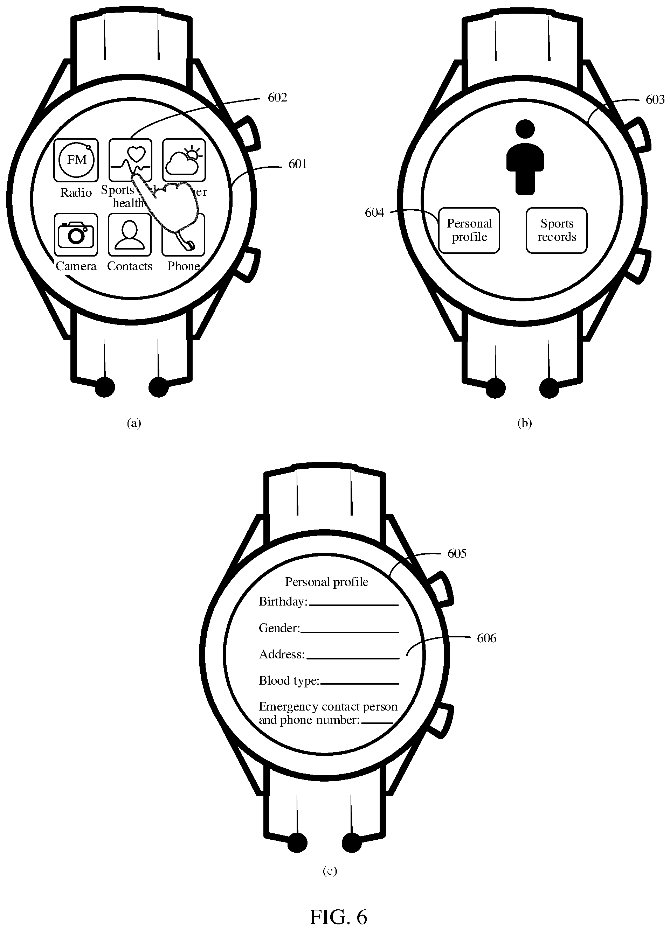

That the first electronic device is a wearable smart device is used as an example. Specifically, a smart watch is used as an example. As shown in (a) in FIG. 6, the smart watch can receive a tap operation of a user on an icon 602 of a Sports and health APP in a home screen interface (namely, a desktop) 601 of the smart watch, and when the Sports and health APP is not running, the mobile phone can start the Sports and health APP in response to the tap operation. An interface 603 shown in (b) in FIG. 6 is also displayed. The interface 603 may include a personal profile function button 604. In response to an operation of the user on the personal profile button 604 on the interface 603, the mobile phone may display an interface 605 as shown in (c) in FIG. 6. The interface 605 may include a personal profile interface 606. The personal profile interface 606 includes personal information like a birthday, a gender, an address, a blood type, an emergency contact, and a phone number. The user may refine the personal information in the personal profile interface 606.

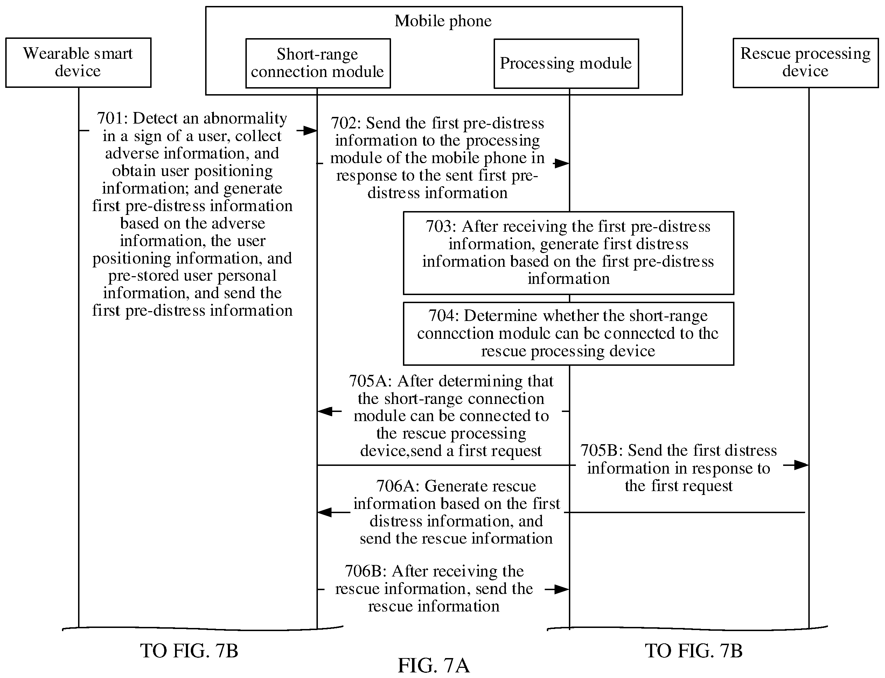

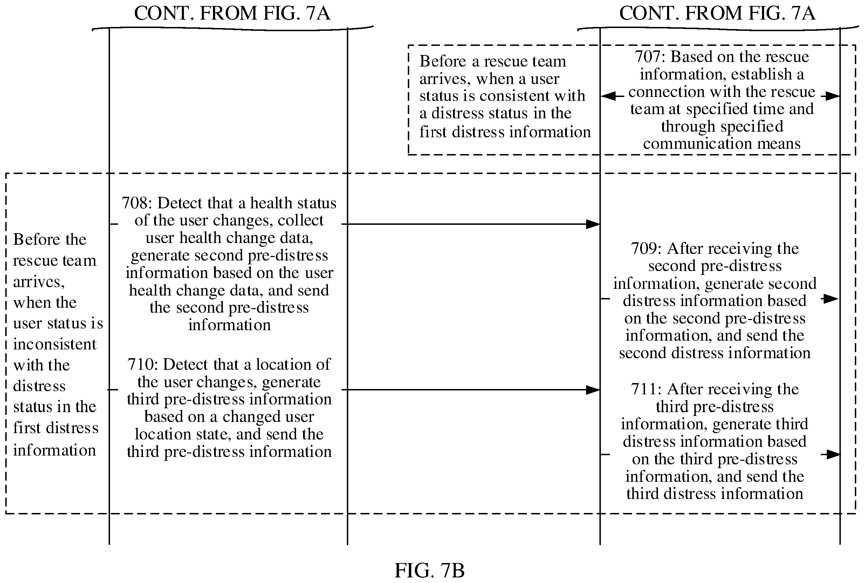

For example, in FIG. 7A and FIG. 7B, a distress information sending method provided by an embodiment of this application is described below, including:
701: A wearable smart device detects an abnormality in a sign of a user, collects adverse information, and obtains user positioning information; and generates first pre-distress information based on the adverse information, the user positioning information, and pre-stored user personal information, and sends the first pre-distress information to a short-range connection module of a mobile phone.

IWith reference to FIG. 1, the user 101 conducts the field research expedition in the deep mountain. When the user 101 is accidentally bitten by the poisonous snake 102, the wearable smart device can detect a blood pressure drop of the user 101. When a blood pressure drop exceeds a preset threshold, the wearable smart device considers that the user 101 currently has an adverse sign and collects adverse sign information including the blood pressure drop.

The wearable smart device automatically sends the first pre-distress information to the short-range connection module of the mobile phone. The first pre-distress information may include the adverse sign information (for example, a blood pressure drop value). In some embodiments, the first pre-distress information further includes at least one of positioning information and personal information. The personal information may be input by the user in the wearable smart device in advance.

Alternatively, when the user 101 perceives that the user is currently in a disaster environment (for example, being bitten by the poisonous snake), the user may manually input the first pre-distress information including the adverse information, the positioning information, and the personal information into the wearable smart device. After receiving the first pre-distress information input by the user, the wearable smart device sends the first pre-distress information to the short-range connection module (the mobile phone and the wearable smart device have a communication connection through the short-range connection module) of the mobile phone.

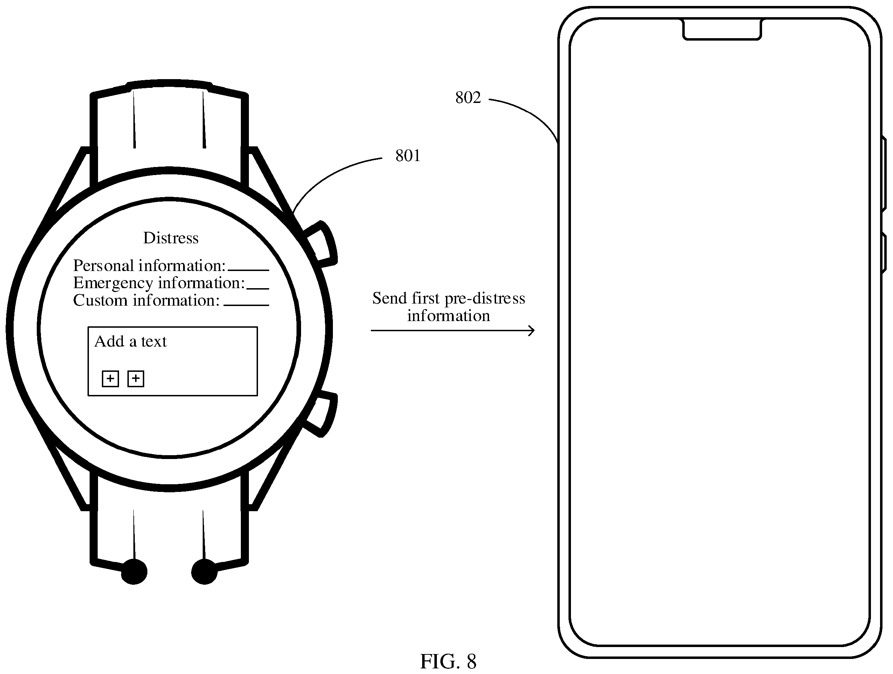

For example, as shown in FIG. 8, when a user encounters a disaster, the user may add first pre-distress information including personal information, emergency information, and custom information of the user to a rescue function in the Sports and health APP of a wearable smart device 801. The custom information may also be chosen to add any one or more of a text, a picture, and a video. Then the wearable smart device 801 sends the obtained first pre-distress information to a mobile phone 802. For example, the wearable smart device 801 may send the obtained first pre-distress information to the mobile phone 802 through a short-range connection module. Subsequently, the wearable smart device 801 may also perform other information exchange with the mobile phone 802 through the short-range connection module during a working process. This is not limited in embodiments of this application. In addition, the Sports and health APP can also be referred to as a Health APP, a Health assistant APP, and the like. This is not limited in embodiments of this application.

Alternatively, when the user 101 perceives that the user is currently in a disaster environment (for example, being bitten by the poisonous snake), the user may press a one-touch rescue function of the wearable smart device, and after receiving a press operation of the user, the wearable smart device sends the first pre-distress information to the short-range connection module of the mobile phone.

Alternatively, the mobile phone is provided with a sensor configured to monitor user sign information. When a change of the user sign information is detected, the sensor may directly send the user sign information to a processing module. The sensor configured to monitor user sign information may include a blood oxygen and heart rate sensing sensor, an optical sensor, and the like. The blood oxygen and heart rate sensing sensor may be configured to monitor blood oxygen and a heart rate. The optical sensor may be configured to monitor a human body heart rate, blood, and other biological characteristics.

Alternatively, when the user 101 perceives that the user is currently in a disaster environment (for example, being bitten by the poisonous snake), the user may manually input the first pre-distress information including the adverse information, the positioning information, and the personal information into the mobile phone in time, and a user interaction module of the mobile phone sends the first pre-distress information to the processing module after receiving the first pre-distress information input by the user.

AIn addition, when the user 101 perceives that the user is currently in the disaster environment (for example, being bitten by the poisonous snake), the user may press the one-button rescue function of the mobile phone, and the mobile phone sends a distress request of the user to the processing module after receiving the pressing operation of the user.

For example, the adverse information presented by the user may include a change in blood pressure that does not meet a preset threshold, a drop or rise in a body temperature that does not meet a preset threshold, and a quantity of times of heartbeats that do not meet a preset threshold for a fixed period of time.

The first pre-distress information includes sign information, positioning information, personal information, emergency information, situated environment information and user custom information.

The sign information may include a blood type, blood pressure, blood oxygen content, a heart rate, and the like.

The positioning information may be determined based on global navigation satellite system GNSS position information of the wearable smart device obtained by the wearable smart device, or may be determined based on GNSS position information of the mobile phone obtained by the positioning module of the mobile phone after the wearable smart device sends the first pre-distress information to the mobile phone. The GNSS may include a global positioning system GPS, a global navigation satellite system GLONASS, a Beidou navigation satellite system BDS, a quasi-zenith satellite system QZSS, or a satellite based augmentation system SBAS. The situated environment information includes a picture of a situated environment, a video of the situated environment, and the like.

The personal information may include a gender, an age, a birthday, and an ID number.

The emergency information may include: a home address, an allergic reaction, an emergency contact, an emergency contact phone number, and the like.

The user custom information may include a brief description of a current situation provided by the user, as well as other information that needs to be notified to the rescue processing device.

In some embodiments, the short-range connection module may include a variety of short-range communication components such as a Wi-Fi component, a Bluetooth component, a Zigbee protocol (Zigbee) component, and an infrared component. The mobile phone may communicate with the wearable smart device by using these short-range communication components. For example, the wearable smart device and the mobile phone may be connected directly through Wi-Fi or join a same hotspot device connection.

Alternatively, the wearable smart device may establish a connection with a Bluetooth component in the short-range connection module of the mobile phone through Bluetooth, and then send the first pre-distress information to the mobile phone through Bluetooth.

Alternatively, the wearable smart device establishes a connection with the Zigbee component in the short-range connection module of the mobile phone through Zigbee, and then sends the first pre-distress information to the mobile phone through Zigbee.

A Zigbee technology is a bidirectional wireless communication technology with a short range, low complexity, low power consumption, a low speed, and low costs, and is mainly used for data transmission between various electronic devices at a short distance, with low power consumption, and a low transmission rate.

702: The short-range connection module of the mobile phone sends the first pre-distress information to the processing module of the mobile phone in response to the first pre-distress information sent by the wearable smart device.

After the short-range connection module of the mobile phone receives the first pre-distress information sent by the wearable smart device, because the short-range connection module does not have a data processing capability, the short-range connection module sends the first pre-distress information to the processing module of the mobile phone.

703: After receiving the first pre-distress information, the processing module of the mobile phone generates first distress information based on the first pre-distress information.

After receiving the first pre-distress information, the processing module may extract content of the first pre-distress information, and then supplement the extracted content to a fixed format of the first distress information.

Alternatively, because there are many types of natural disasters, the processing module extracts content of the first pre-distress information, and analyzes the extracted content after extraction. Then, based on an analysis result, the content of the first pre-distress information is sorted based on an importance degree. Information that is sorted at the forefront is marked for processing, and the marked sorting information is used as the first distress information. In this way, after the rescue processing device receives the first distress information, the rescue processing device may quickly determine important information based on the sorting and a mark, and prepare required supplies for a distressed person based on the important information.

For example, with reference to FIG. 1, after the user 101 is bitten by the poisonous snake 102, the wearable smart device sends the first pre-distress information to the mobile phone. The first pre-distress information includes indication information that a current blood pressure drop of the user exceeds a threshold, cause explanation information of the blood pressure drop, and the like. The cause explanation information of the blood pressure drop may be a user custom input field. After receiving the first pre-distress information, the mobile phone may adjust sorting of the indication information that a current blood pressure drop of the user exceeds a threshold and the cause explanation information of the blood pressure drop to the front, and then mark the indication information that a current blood pressure drop of the user exceeds a threshold and the cause explanation information of the blood pressure drop. Finally, the marked sorting information is sent to the rescue processing device as the first distress information. This is convenient for the rescue processing device to process the first distress information in time after receiving the first distress information.

For example, the first distress information may include identity information of a distressed person, location information of the distressed person, physical health data such as a blood type, blood pressure, blood oxygen content, a heart rate, and the like of the distressed person, an emergency contact and contact information set by the distressed person, a photo and a video of the environment in which the distressed person is situated, a text, an image, an audio or a video customized by the distressed person, and the like.

704: The processing module of the mobile phone determines whether the short-range connection module can be connected to the rescue processing device.

The mobile phone may perform a network connection test by invoking a packet Internet groper (Packet Internet Groper, PING) program, and determine whether the short-range connection module can be connected to the rescue processing device based on a network connection test result.

Specifically, The PING sends an Internet control messages protocol (Internet Control Messages Protocol, ICMP) echo request message to the rescue processing device by using a communication network provided by the short-range connection module and reports a desired ICMP echo response. In preset time, when the desired ICMP echo response is received, it is considered that the mobile phone can be connected to the rescue processing device by using the short-range connection module. Alternatively, when the desired ICMP echo response is not received, it is considered that the mobile phone cannot be connected to the rescue processing device by using the short-range connection module.

In some embodiments, the short-range connection module may include a first short-range connection and a second short-range connection module. The first short-range connection module is configured to connect the wearable smart device and the mobile phone, and the second short-range connection module is configured to connect the mobile phone and the rescue processing device. The first short-range connection module may include a variety of short-range communication components such as a Wi-Fi component, a Bluetooth component, and a Zigbee component. The second short-range connection module may also include a variety of short-range communication components such as a Wi-Fi component, a Bluetooth component, and a Zigbee component.

The mobile phone may communicate with the wearable smart device by using the first short-range connection module. Specifically, the mobile phone may communicate with the wearable smart device by using the Wi-Fi component in the first short-range connection module, may alternatively communicate with the wearable smart device by using the Bluetooth component in the first short-range connection module, or may communicate with the wearable smart device by using the Zigbee component in the first short-range connection module.

The mobile phone may communicate with the rescue processing device by using the second short-range connection module. Specifically, the mobile phone may communicate with the rescue processing device by using the Wi-Fi component in the second short-range connection module, may alternatively communicate with the rescue processing device by using the Bluetooth component in the second short-range connection module, or may communicate with the rescue processing device by using the Zigbee component in the second short-range connection module.

Components in the second short-range connection module used for communication between the mobile phone and the rescue processing device may be the same as components in the first short-range connection module used for communication between the mobile phone and the wearable smart device. That is, the mobile phone communicates with the rescue processing device by using the Wi-Fi component, and communicates with the wearable smart device by using the Wi-Fi component.

Alternatively, the mobile phone communicates with the rescue processing device by using the Bluetooth component, and communicates with the wearable smart device by using the Bluetooth component.

Alternatively, the mobile phone communicates with the rescue processing device by using the Zigbee component, and communicates with the wearable smart device by using the Zigbee component.

Components in the second short-range connection module used for communication between the mobile phone and the rescue processing device may be different from components in the first short-range connection module used for communication between the mobile phone and the wearable smart device. That is, the mobile phone communicates with the rescue processing device by using the Wi-Fi component, and communicates with the wearable smart device by using the Bluetooth component (or the Zigbee component).

Alternatively, the mobile phone communicates with the rescue processing device by using the Bluetooth component and communicates with the wearable smart device by using the Wi-Fi component (or the Zigbee component).

Alternatively, the mobile phone communicates with the rescue processing device by using the Zigbee component and communicates with the wearable smart device by using the Wi-Fi component (or the Bluetooth component).

In some other embodiments, the short-range connection module may include a variety of short-range communication components such as a Wi-Fi component, a Bluetooth component, and a Zigbee component, and the mobile phone may communicate with the wearable smart device and the rescue processing device separately by using short-range communication components in the short-range communication module.

Specifically, a short-range communication component in the short-range connection module used for communication between the mobile phone and the wearable smart device and a short-range communication component in the short-range connection module used for communication between the mobile phone and the rescue processing device may be the same or different.

705A: After determining that the short-range connection module can be connected to the rescue processing device, the processing module of the mobile phone sends a first request to the short-range connection module, where the first request includes the first distress information.

The first request is used for requesting to send the first distress information to the rescue processing device.

For example, the processing module may send the first request to the short-range connection module. With reference to 704, specifically, the processing module sends the first request to the second short-range connection module. Because the second short-range connection module includes the variety of short-range communication components such as the Wi-Fi component, the Bluetooth component, and the Zigbee component, The processing module of the mobile phone sends the first request to the rescue processing device by using a short-range communication component capable of communicating with the rescue processing device.

705B: The short-range connection module of the mobile phone responds to the first request and sends the first distress information to the rescue processing device.

706A: The rescue processing device generates rescue information based on the first distress information, and sends the rescue information to the short-range connection module of the mobile phone.

IWith reference to 705A, the short-range connection module of the mobile phone can be connected to rescue processing device. Usually, after the mobile phone sends the first distress information, the short-range connection module of the mobile phone may also receive the rescue information sent by the rescue processing device. When the short-range connection module is abnormal and the mobile phone cannot receive the rescue information sent by the rescue processing device within preset time after sending out the first distress information, the mobile phone needs to switch to other communication means for distress. The other communication means may be cellular communication, satellite communication, communication between remote devices, and the like.

706B: After receiving the rescue information, the short-range connection module of the mobile phone sends the rescue information to the processing module of the mobile phone.

Usually, after the rescue processing device receives the first distress information sent by using the short-range connection module, the rescue processing device first contacts a rescue team closest to the distressed person to determine whether there is time to provide rescue for the distressed person based on a sending location of the first distress information. After confirming that the rescue team can provide rescue for the distressed person, the rescue processing device informs the rescue team of a basic situation of the distressed person and negotiates a specific rescue plan. After the rescue plan is determined, the rescue processing device notifies the rescue team to execute the rescue plan, and at the same time sends the rescue information to the mobile phone. In this way, it is convenient for the rescue team to contact the distressed person as soon as possible through a manner agreed in the rescue information upon arrival.

In some embodiments, the rescue processing device usually feeds back the rescue information to the distressed person based on communication means of the received first distress information. Therefore, the short-range connection module of the mobile phone may receive the rescue information sent from the rescue processing device, and then the short-range connection module sends the rescue information to the processing module. The processing module displays the received rescue information to the user through a display of the mobile phone to inform the user of a current rescue progress.

For example, the rescue information may include estimated arrival time of the rescue team, communication means between the rescue team and the distressed person, a forecast of weather information near a distress location of the distressed person, a preliminary emergency suggestion provided for the first distress information, text information, image information, audio information, or video information that is customized by the rescue team, and text information, image information, audio information, or video information that is fed back by an emergency contact of the distressed person, and the like.

The communication means between the rescue team and the distressed person included in the rescue information may be communication means adopted by the distressed person to send the first distress information. To ensure that rescue can be received more reliably, the rescue processing device may send the rescue information through various communication means, such as short-range connection communication, cellular network communication, satellite communication, and communication between remote devices.

A communication network between remote devices is a decentralized communication network established by the terminal device and the rescue processing device by using a preset frequency resource. The preset frequency resource includes an unauthorized frequency resource. Further, the preset frequency resource is a narrowband frequency resource.

A data type transmitted by the communication network between remote devices includes at least one of a picture, a text, a video, or voice.

For example, the communication network between remote devices may be a point-to-point communication network established by the mobile phone and the rescue processing device by using the preset frequency resource. The preset frequency resource may be a preset unauthorized frequency resource (for example, a resource other than a frequency resource used by an operator). Further, the preset frequency resource may be a narrowband frequency resource.

Because the mobile phone and the rescue processing device do not need to pass through any central device (for example, a base station device) when communicating with each other by using the communication network between remote devices, the communication network between remote devices may also be regarded as a decentralized communication network established by the mobile phone and the rescue processing device by using the preset frequency resource.

After a communication link is established by using the communication network between remote devices, many types of data, such as a picture, a text, a video, or voice, can be transmitted between the mobile phone and the rescue processing device.

A reason why the distressed person sends the first distress information is usually because the distressed person is located in a location with harsh environment and incomplete basic communication facilities like the wild, a disaster area, or a mountain. Therefore, the distressed person can be notified in the rescue information, and a communication function between remote devices can be turned on at a specified time point. Then, through the communication function between remote devices, the rescue team can find the distressed person in time and establish a rescue link in a process of approaching the distressed person.

Before the rescue team arrives, when a user status is consistent with a distress status in the first distress information, the following 707 is performed.

Specifically, a state described by user information, the user positioning information and the adverse sign information of the user in the first distress information is the distress status.

707: The processing module of the mobile phone establishes a connection with the rescue team based on the rescue information at specified time and through specified communication means.

After receiving the rescue information, the processing module analyzes the rescue information, and then establishes the rescue link with the rescue team through the communication means indicated by the rescue information based on the time point indicated in the rescue information. When the communication means indicated by the rescue information is not available, the processing module may attempt to establish a connection with the rescue team by using a module for communication between remote devices.

When the rescue link is successfully established, the distressed person can wait for rescue of the rescue team.

When the rescue link fails to be established, the processing module sends the first distress information to the rescue processing device again until the rescue link is successfully established. The re-sent first distress information may be sent by using any one of the short-range connection module, the cellular communication module, the satellite communication module, or the module for communication between remote devices.

Before the rescue team arrives, when the user status is inconsistent with the distress status in the first distress information, the following 708 to 711 are performed.

That the user status is inconsistent with the distress status in the first distress information may be specifically that user adverse sign information obtained when the user waits for rescue of the rescue team is inconsistent with the user adverse sign information in the first pre-distress information, may alternatively be that positioning information obtained when the user waits for rescue of the rescue team is inconsistent with the user positioning information in the first pre-distress information, or that the positioning information obtained when the user waits for rescue of the rescue team is inconsistent with the user positioning information in the first distress information.

708: The wearable smart device detects that a health status of the user changes, collects user health change data, generates second pre-distress information based on the user health change data, and sends the second pre-distress information to the processing module of the mobile phone by using the short-range connection module.

When the user waits for the rescue of the rescue team, the wearable smart device still monitors the status of the user. When the wearable smart device monitors that the health status of the user changes and a change interval exceeds a threshold, the wearable smart device re-generates the second pre-distress information based on latest changed health status of the user, to implement accurate rescue.

Alternatively, when the user perceives that the health status of the user changes, the user may manually input change information of the health status in the wearable smart device. After the wearable smart device receives the change information of the health status input by the user, the wearable smart device re-generates the second pre-distress information based on the change information of the health status.

Alternatively, the mobile phone is provided with a sensor for monitoring the health status of the user. When detecting that the health status of the user changes, the sensor may send change information of the health status of the user to the processing module, and the processing module re-generates the second pre-distress information based on the change information of the health status of the user.

Alternatively, when the user perceives that currently the health status of the user changes, the user may manually input second pre-distress information including the changed health status into the mobile phone in time, and the user interaction module of the mobile phone sends the second pre-distress information to the processing module after receiving the second pre-distress information.

709: After receiving the second pre-distress information, the processing module of the mobile phone generates second distress information based on the second pre-distress information, and re-sends the second distress information to the rescue processing device.

For the part in which the processing module generates the second distress information based on the second pre-distress information, refer to a manner of generating the first distress information from the first pre-distress information, and refer to 703 for details. After generating the second distress information, the processing module may sequentially try to send the second distress information to the rescue processing device by using the short-range connection module, the cellular communication module, the satellite communication module, and the module for communication between remote devices until the second distress information is successfully sent. After the second distress information is sent, 706A to 711 may be continued.

710: The wearable smart device detects that a location of the user changes, generates third pre-distress information based on a changed user location state, and sends the third pre-distress information to the processing module of the mobile phone by using the short-range connection module.

When the user waits for the rescue of the rescue team, the wearable smart device still monitors the status of the user. When the wearable smart device detects that the location of the user changes and a change interval exceeds a threshold, the wearable smart device re-generates the third pre-distress information based on latest changed location of the user, to implement accurate rescue.

Alternatively, with reference to FIG. 1, when the user 101 waits for rescue, it appears that there is another person ahead. Then the user 101 goes in a direction of the another person to try to get help from the another person. But after walking for a while, the user 101 did not keep up with the another person. Because the user 101 has a poor sense of direction in the deep mountain and cannot return to a place where the first distress information is sent, the user 101 can only manually input the changed location information in the wearable smart device. After the wearable smart device receives the changed location information input by the user 101, the wearable smart device re-generates the third pre-distress information based on the changed location information.

Alternatively, the wearable smart device periodically detects the location of the user 101 at a fixed period. When it is detected that a location of the user 101 changes, detection is started all the time. When a moving speed of the user 101 is 0 and does not change in preset time, the wearable smart device determines that the location of the user 101 will not change, then the wearable smart device re-detects a location and generates the third pre-distress information based on the latest detected location.

Alternatively, the mobile phone is provided with a positioning module for monitoring the location of the user. When it is detected that the location of the user changes, the positioning module may re-detect the location of the user and send the detected newest location to the processing module. The processing module generates fourth distress information based on the detected newest location and sends the fourth distress information to the rescue processing device.

711: After receiving the third pre-distress information, the processing module of the mobile phone generates third distress information based on the third pre-distress information, and sends the third distress information to the rescue processing device.

For the part in which the processing module generates the third distress information based on the third pre-distress information, refer to a manner of generating the first distress information from the first pre-distress information, and refer to 703 for details. After generating the third distress information, the processing module can sequentially try to send the second distress information to the rescue processing device by using the short-range connection module, the cellular communication module, the satellite communication module, and the module for communication between remote devices. After the third distress information is sent, 706A to 711 may be continued.

Usually, use costs of a short-range connection module, a cellular communication module, and a satellite communication module increase in sequence, and a module for communication between remote devices requires that two devices turn on this function at the same time to implement communication. This is difficult to use. Therefore, a communication manner in which communication is performed by using the short-range connection module first, and when communication fails, communication is performed by using the cellular communication module, the satellite communication module, and the module for communication between remote devices in sequence. This can reduce use costs, and ensure smooth sending of distress information as much as possible, so that a distress seeker can be rescued in time.

It should be noted that, embodiments of this disclosure may not limit an execution sequence between 708 and 709 and 710 and 711. For example, 708 and 709 may be performed first, followed by 710 and 711. Alternatively, 710 and 711 may be performed first, followed by 708 and 709. Alternatively, 708 and 709 and 710 and 711 may be performed at the same time. Alternatively, only 708 and 709 are performed first, and 710 and 711 are not performed. Alternatively, only 710 and 711 are performed, and 708 and 709 are not performed. This may be specifically determined based on an actual use requirement.

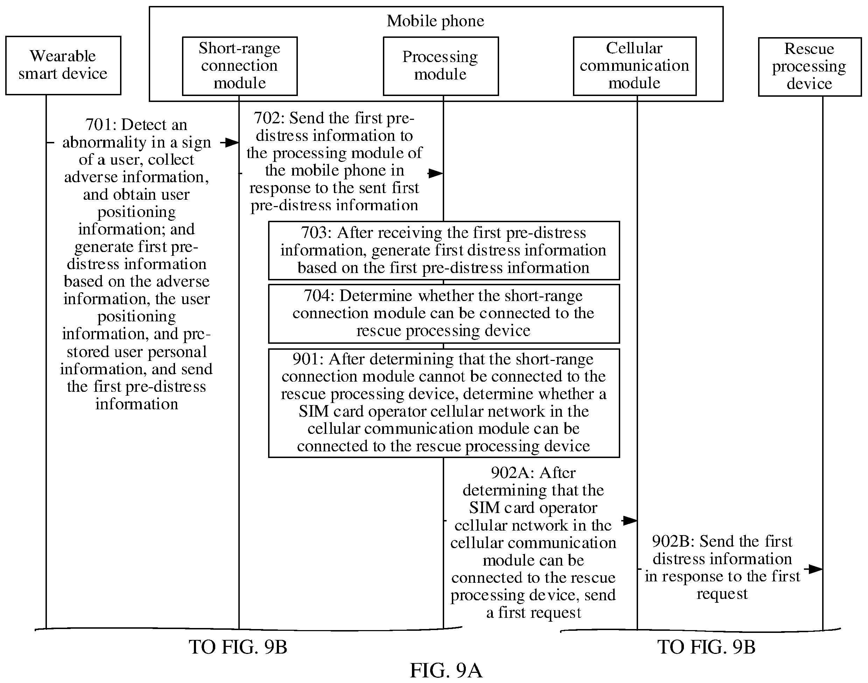

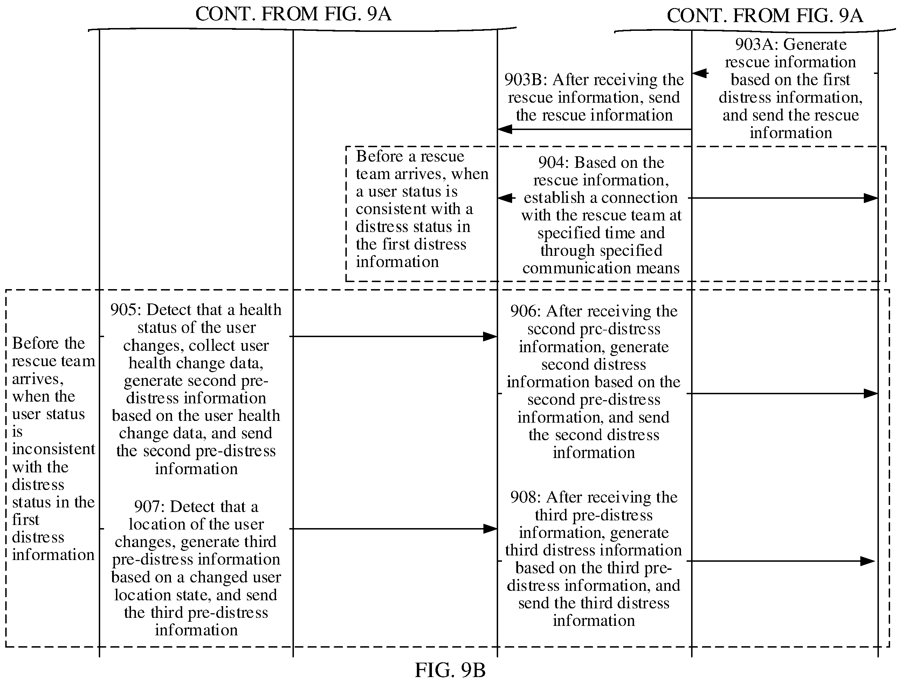

For example, in FIG. 9A and FIG. 9B, another distress information sending method provided by an embodiment of this application is described below. After 701 to 704, the method further includes:
901: After determining that the short-range connection module cannot be connected to the rescue processing device, the processing module of the mobile phone determines whether a SIM card operator cellular network in the cellular communication module can be connected to the rescue processing device.

When the mobile phone is in normal use, at least one SIM card is installed on the mobile phone. The processing module of the mobile phone determines whether the SIM card operator cellular network in the cellular communication module can be connected to the rescue processing device, and specifically, the processing module of the mobile phone determines whether an operator cellular network corresponding to each SIM card can be connected to the rescue processing device.

A specific process in which the processing module of the mobile phone determines whether the SIM card operator cellular network in the cellular communication module can be connected to the rescue processing device is as follows: The cellular communication module of the mobile phone obtains a signal strength measurement result and a resident status of the operator cellular network corresponding to each SIM card. When the operator cellular network corresponding to the SIM card successfully resides in a current area, and the signal strength measurement result is greater than or equal to a preset threshold, it is considered that the SIM card operator cellular network in the cellular communication module can be connected to the rescue processing device. When the operator cellular network corresponding to the SIM card cannot successfully reside in a current area, or when the operator cellular network corresponding to the SIM card can successfully reside in the current area, but the signal strength measurement result is less than a preset threshold, the SIM card operator cellular network in the cellular communication module cannot be connected to the rescue processing device.

When the SIM card operator cellular network in the cellular communication module of the mobile phone can be connected to the rescue processing device, the first distress information can be sent to the rescue processing device by using the cellular communication module of the mobile phone. The first distress information can be sent in a form of a data service, can be sent in a form of a call, or can be sent in a form of a short message. Therefore, the set preset threshold can meet a network quality requirement of the data service, the call, or the short message.

In some embodiments, the cellular communication module may establish a connection with the rescue processing device by using a SIM card operator cellular network. When the mobile phone used by the distressed person has two SIM cards, the processing module can respectively determine whether the operator cellular network corresponding to each SIM card can be connected to the rescue processing device.

For example, when the SIM cards of the mobile phone used by the distressed person are a Mobile SIM card and a Unicom SIM card, the processing module can first determine whether the processing module can be connected to the rescue processing device by using a Mobile network, and then determine whether the processing module can be connected to the rescue processing device by using a Unicom network. Alternatively, the processing module can first determine whether the processing module can be connected to the rescue processing device by using a Unicom network, and then determine whether the processing module can be connected to the rescue processing device by using a Mobile network. Specific determining sequence is not limited in this application.

When the mobile phone used by the distressed person has only one SIM card, the processing module can directly determine whether an operator cellular network corresponding to the SIM card can be connected to the rescue processing device.

902A: After determining that the SIM card operator cellular network in the cellular communication module can be connected to the rescue processing device, the processing module of the mobile phone sends a first request to the cellular communication module.

The first request is used for requesting to send the first distress information to the rescue processing device. The first request includes the first distress information.

902B: The cellular communication module of the mobile phone sends the first distress information to the rescue processing device in response to the first request.

903A: The rescue processing device generates rescue information based on the first distress information, and sends the rescue information to the cellular communication module of the mobile phone.

903B: After receiving the rescue information, the cellular communication module of the mobile phone sends the rescue information to the processing module of the mobile phone.

Usually, after the rescue processing device receives the first distress information sent by using the SIM card operator cellular network in the cellular communication module, the rescue processing device contact a rescue team closest to the distressed person to provide rescue for the distressed person based on a sending location of the first distress information. After the rescue processing device determines a corresponding rescue plan and rescue team, the rescue processing device sends rescue information to the mobile phone.

Specifically, the rescue processing device feeds back the rescue information to the distressed person based on communication means of the received first distress information. Therefore, the SIM card operator cellular network in the cellular communication module of the mobile phone can receive the rescue information sent from the rescue processing device, and then the cellular communication module sends the rescue information to the processing module. The processing module displays the received rescue information to the user through a display of the mobile phone to inform the user of a current rescue progress.

Before the rescue team arrives, when a user status is consistent with a distress status in the first distress information, the following 904 is performed.

904: Based on the rescue information, the processing module of the mobile phone establishes a connection with the rescue team at specified time and through specified communication means.

After receiving the rescue information, the processing module analyzes the rescue information, and then establishes the rescue link with the rescue team through the communication means indicated by the rescue information based on the time point indicated in the rescue information. The rescue processing device usually indicates communication means between the rescue team and the distressed person based on the communication means of the received first distress information.

Therefore, the processing module can establish the rescue link with the rescue team by using the SIM card operator cellular network in the cellular communication module. When the SIM card operator cellular network in the cellular communication module indicated by the rescue information is not available, the processing module may further attempt to establish a connection with the rescue team by using the module for communication between remote devices.

When the rescue link is successfully established, the distressed person can wait for rescue of the rescue team.

When the rescue link fails to be established, the processing module sends the first distress information to the rescue processing device again until the rescue link is successfully established. The re-sent first distress information may be sent by using any one of the short-range connection module, the cellular communication module, the satellite communication module, or the module for communication between remote devices.

Before the rescue team arrives, when the user status is inconsistent with the distress status in the first distress information, the following 905 to 908 are performed.

905: The wearable smart device detects that a health status of the user changes, collects user health change data, generates second pre-distress information based on the user health change data, and sends the second pre-distress information to the processing module of the mobile phone by using the short-range connection module.

Refer to 709 for details.

906: After receiving the second pre-distress information, the processing module of the mobile phone generates second distress information based on the second pre-distress information, and sends the second distress information to the rescue processing device.

For the part in which the processing module generates the second distress information based on the second pre-distress information, refer to a manner of generating the first distress information from the first pre-distress information, and see 703 for details. After generating the second distress information, the processing module can sequentially try to send the second distress information to the rescue processing device by using the short-range connection module, the cellular communication module, the satellite communication module, and the module for communication between remote devices. After the second distress information is sent, 903A to 908 can be continued.

907: The wearable smart device detects that a location of the user changes, generates third pre-distress information based on a changed user location state, and sends the third pre-distress information to the processing module of the mobile phone by using the short-range connection module.

Refer to 710 for details.

908: After receiving the third pre-distress information, the processing module of the mobile phone generates third distress information based on the third pre-distress information, and sends the third distress information to the rescue processing device.

For the part in which the processing module generates the third distress information based on the third pre-distress information, refer to a manner of generating the first distress information from the first pre-distress information, and see 703 for details. After generating the third distress information, the processing module can sequentially try to send the second distress information to the rescue processing device by using the short-range connection module, the cellular communication module, the satellite communication module, and the module for communication between remote devices. After the third distress information is sent, 903A to 908 can be continued.

It should be noted that, embodiments of this disclosure may not limit an execution sequence between 905 and 906 and 907 and 908. For example, 905 and 906 may be performed first, followed by 907 and 908. Alternatively, 907 and 908 may be performed first, followed by 905 and 906. Alternatively, 905 and 906 and 907 and 908 may be performed at the same time. Alternatively, only 905 and 906 are performed first, and 907 and 908 are not performed. Alternatively, only 907 and 908 are performed, and 905 and 906 are not performed. This may be specifically determined based on an actual use requirement.

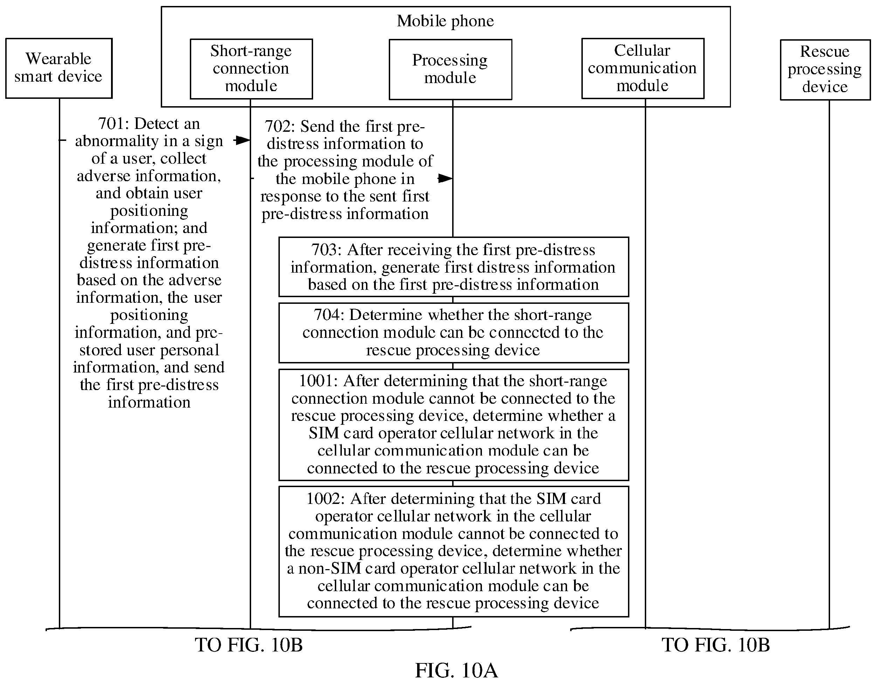

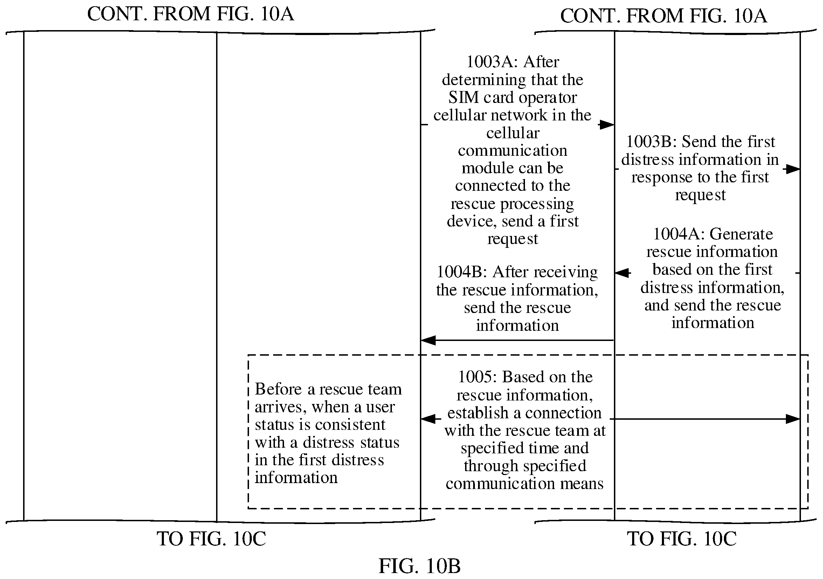

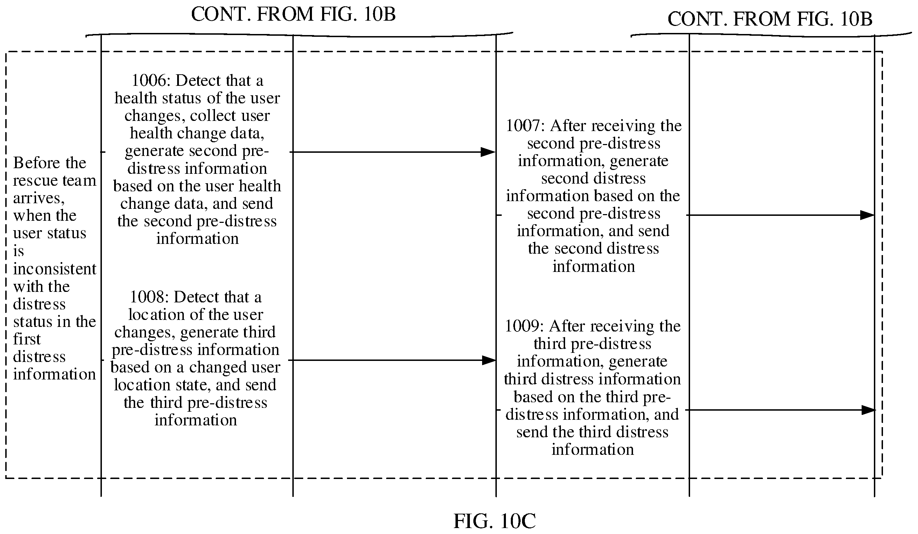

For example, in FIG. 10A to FIG. 10C, another distress information sending method provided by an embodiment of this application is described below. After 701 to 704, the method further includes:
1001: After determining that the short-range connection module cannot be connected to the rescue processing device, the processing module of the mobile phone determines whether a SIM card operator cellular network in the cellular communication module can be connected to the rescue processing device.

Specifically, when the SIM card operator cellular network in the cellular communication module can be connected to the rescue processing device, the processing module can directly send the first distress information to the rescue processing device by using the SIM card operator cellular network in the cellular communication module without continuing to perform 1002 and 1003A.

1002: After determining that the SIM card operator cellular network in the cellular communication module cannot be connected to the rescue processing device, the processing module of the mobile phone determines whether a non-SIM card operator cellular network in the cellular communication module can be connected to the rescue processing device.

In some embodiments, when the operator cellular network corresponding to the SIM card installed in the mobile phone cannot be connected to the rescue processing device, it may continue to determine whether the non-SIM card operator cellular network can be connected to the rescue processing device. The non-SIM card operator cellular network is an operator other than an operator corresponding to the SIM card in the mobile phone.

For example, a non-SIM card operator cellular network may be a Telecom network when there are two SIM cards that are of a mobile phone used by a distressed person and that are a Mobile SIM card and a Unicom SIM card respectively. When only one SIM card of the mobile phone used by the distressed person is a Mobile SIM card, the non-SIM card operator cellular network may be a Telecom network or a Unicom network.

A specific process in which the processing module of the mobile phone determines whether the non-SIM card operator cellular network in the cellular communication module can be connected to the rescue processing device is as follows: The cellular communication module of the mobile phone obtains a signal strength measurement result and a resident status of the non-SIM card operator cellular network. When the non-SIM card operator cellular network successfully resides in a current area, and the signal strength measurement result is greater than a preset threshold, it is considered that the non-SIM card operator cellular network in the cellular communication module can be connected to the rescue processing device.

When the non-SIM card operator cellular network cannot successfully reside in a current area, or when the non-SIM card operator cellular network can successfully reside in the current area, but the signal strength measurement result is less than a preset threshold, the non-SIM card carrier cellular network in the cellular communication module cannot be connected to the rescue processing device.

When the non-SIM card operator cellular network in the cellular communication module of the mobile phone can be connected to the rescue processing device, the first distress information can be sent to the rescue processing device by using the cellular communication module of the mobile phone. The first distress information can only be sent in a form of an emergency call. Therefore, the set preset threshold can meet a network quality requirement of the emergency call.

1003A: After determining that the non-SIM card operator cellular network in the cellular communication module can be connected to the rescue processing device, the processing module of the mobile phone sends a first request to the cellular communication module.

The first request is used for requesting to send the first distress information to the rescue processing device. The first request includes the first distress information.

1003B: The cellular communication module of the mobile phone sends the first distress information to the rescue processing device in response to the first request.

1004A: The rescue processing device generates rescue information based on the first distress information, and sends the rescue information to the cellular communication module of the mobile phone.

1004B: After receiving the rescue information, the cellular communication module of the mobile phone sends the rescue information to the processing module of the mobile phone.

Usually, after the rescue processing device receives the first distress information sent by using the non-SIM card operator cellular network in the cellular communication module, the rescue processing device contact a rescue team closest to the distressed person to provide rescue for the distressed person based on a sending location of the first distress information. After the rescue processing device determines a corresponding rescue plan and rescue team, the rescue processing device sends rescue information to the mobile phone.

Specifically, the rescue processing device feeds back the rescue information to the distressed person based on communication means of the received first distress information.

Therefore, the non-SIM card operator cellular network in the cellular communication module of the mobile phone can receive the rescue information sent from the rescue processing device, and then the cellular communication module sends the rescue information to the processing module. The processing module displays the received rescue information to the user through a display of the mobile phone to inform the user of a current rescue progress.

Before the rescue team arrives, when a user status is consistent with a distress status in the first distress information, the following 1005 is performed.

1005: Based on the rescue information, the processing module of the mobile phone establishes a connection with the rescue team at specified time and through specified communication means.

After receiving the rescue information, the processing module analyzes the rescue information, and then establishes the rescue link with the rescue team through the communication means indicated by the rescue information based on the time point indicated in the rescue information. The rescue processing device usually indicates communication means between the rescue team and the distressed person based on the communication means of the received first distress information.

Therefore, the processing module can establish the rescue link with the rescue team by using the non-SIM card operator cellular network in the cellular communication module. When the non-SIM card operator cellular network in the cellular communication module indicated by the rescue information is not available, the processing module may further attempt to establish a connection with the rescue team by using the module for communication between remote devices.

When the rescue link is successfully established, the distressed person can wait for rescue of the rescue team.

When the rescue link fails to be established, the processing module sends the first distress information to the rescue processing device again until the rescue link is successfully established. The re-sent first distress information may be sent by using any one of the short-range connection module, the cellular communication module, the satellite communication module, or the module for communication between remote devices.

Before the rescue team arrives, when the user status is inconsistent with the distress status in the first distress information, the following 1006 to 1013 are performed.

1006: The wearable smart device detects that a health status of the user changes, collects user health change data, generates second pre-distress information based on the user health change data, and sends the second pre-distress information to the processing module of the mobile phone by using the short-range connection module.

Refer to 708 for details.

1007: After receiving the second pre-distress information, the processing module of the mobile phone generates second distress information based on the second pre-distress information, and sends the second distress information to the rescue processing device.

For the part in which the processing module generates the second distress information based on the second pre-distress information, refer to a manner of generating the first distress information from the first pre-distress information, and see 703 for details.

After generating the second distress information, the processing module can sequentially try to send the second distress information to the rescue processing device by using the short-range connection module, the cellular communication module, the satellite communication module, and the module for communication between remote devices. After the second distress information is sent, 1004A to 1009 can be continued.

1008: The wearable smart device detects that a location of the user changes, generates third pre-distress information based on a changed user location state, and sends the third pre-distress information to the processing module of the mobile phone by using the short-range connection module.

Refer to 710 for details.

1009: After receiving the third pre-distress information, the processing module of the mobile phone generates third distress information based on the third pre-distress information, and sends the third distress information to the rescue processing device.

For the part in which the processing module generates the third distress information based on the third pre-distress information, refer to a manner of generating the first distress information from the first pre-distress information, and see 703 for details.

After generating the third distress information, the processing module can sequentially try to send the second distress information to the rescue processing device by using the short-range connection module, the cellular communication module, the satellite communication module, and the module for communication between remote devices. After the third distress information is sent, 1004A to 1009 can be continued.

It should be noted that, embodiments of this disclosure may not limit an execution sequence between 1006 and 1007 and 1008 and 1009. For example, 1006 and 1007 may be performed first, followed by 1008 and 1009. Alternatively, 1008 and 1009 may be performed first, followed by 1006 and 1007. Alternatively. 1006 and 1007 and 1008 and 1009 may be performed at the same time. Alternatively, only 1006 and 1007 are performed first, and 1008 and 1009 are not performed. Alternatively, only 1008 and 1009 are performed, and 1006 and 1007 are not performed. This may be specifically determined based on an actual use requirement.

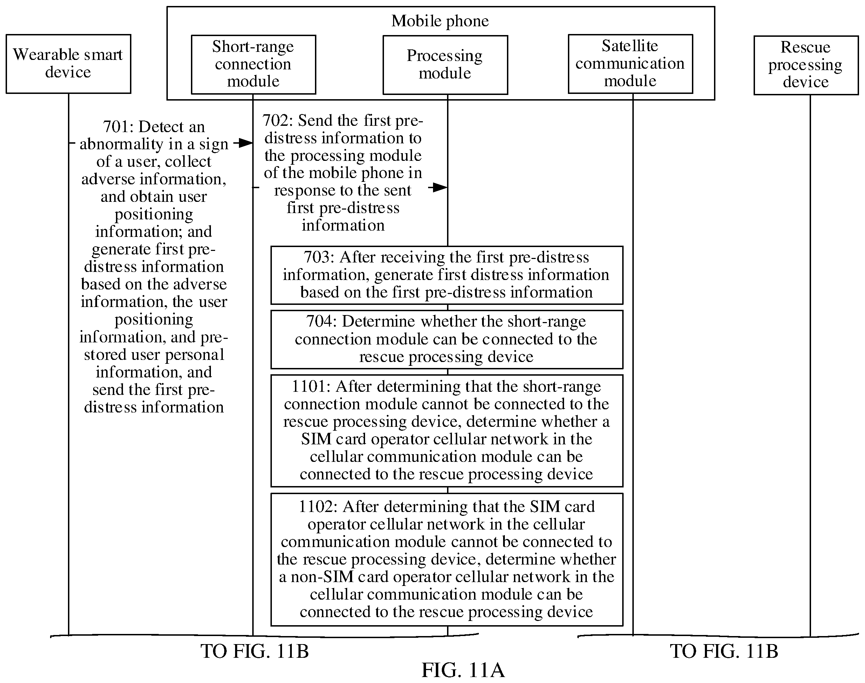

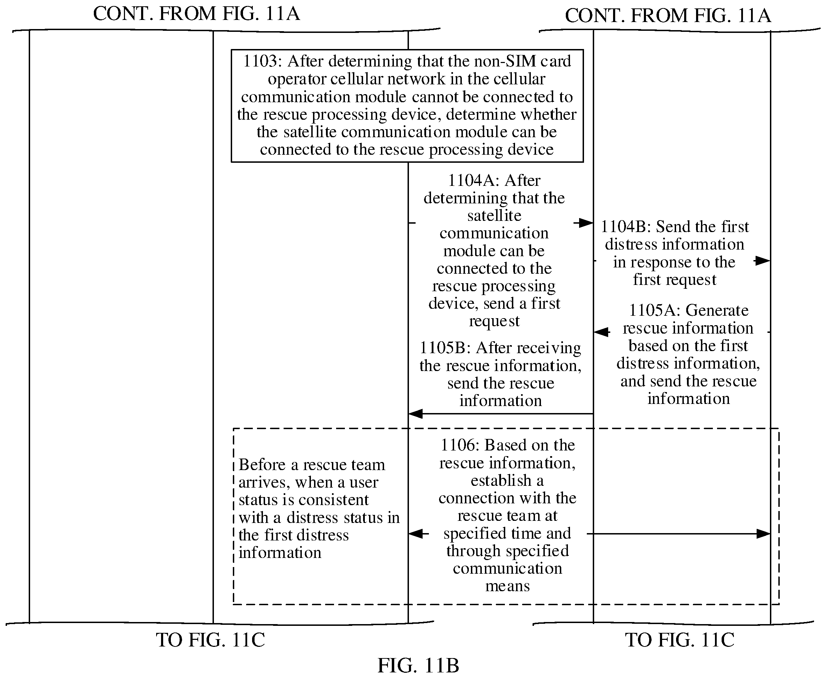

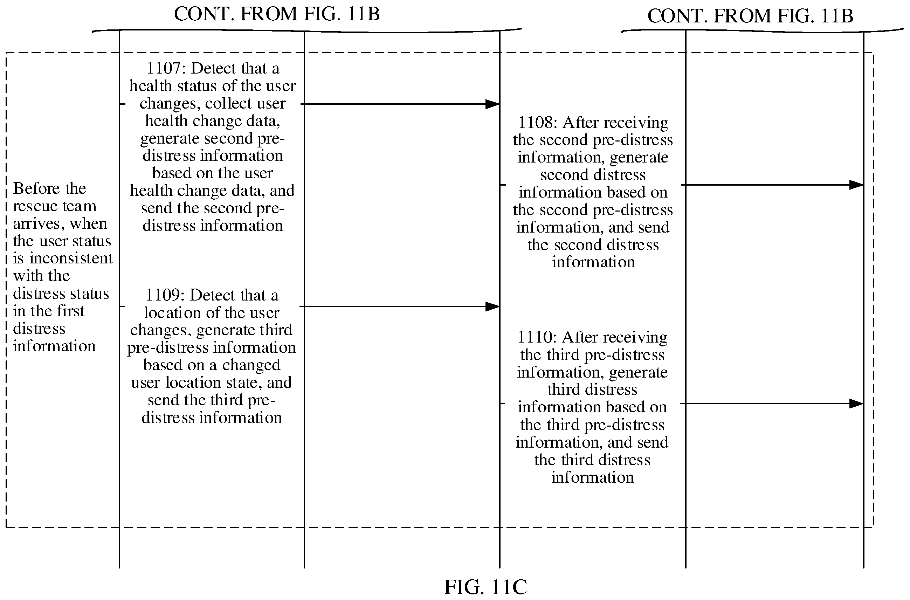

For example, in FIG. 11A to FIG. 11C, another distress information sending method provided by an embodiment of this application is described below. After 701 to 704, the method further includes:
1101: After determining that the short-range connection module cannot be connected to the rescue processing device, the processing module of the mobile phone determines whether a SIM card operator cellular network in the cellular communication module can be connected to the rescue processing device.
   Specifically, when the SIM card operator cellular network in the cellular communication module can be connected to the rescue processing device, the processing module can directly send the first distress information to the rescue processing device by using the SIM card operator cellular network in the cellular communication module without continuing to perform 1102 to 1104A.
1102: After determining that the SIM card operator cellular network in the cellular communication module cannot be connected to the rescue processing device, the processing module of the mobile phone determines whether a non-SIM card operator cellular network in the cellular communication module can be connected to the rescue processing device.

Specifically, when the non-SIM card operator cellular network in the cellular communication module can be connected to the rescue processing device, the processing module can directly send the first distress information to the rescue processing device by using the non-SIM card operator cellular network in the cellular communication module without continuing to perform 1102 to 1104A.

1103: After determining that the non-SIM card operator cellular network in the cellular communication module cannot be connected to the rescue processing device, the processing module of the mobile phone determines whether the satellite communication module can be connected to the rescue processing device.

A process of determining whether the satellite communication module can be connected to the rescue processing device by the processing module is to be supplemented.

The mobile phone can obtain the signal strength measurement result of the satellite communication network and the resident status of the satellite communication network by using the satellite communication module, and then determine whether the satellite communication module can send the first distress information based on the signal strength measurement result and the resident status.

Specifically, when the satellite communication network resides in the current area and the signal strength measurement result exceed a preset threshold, it is considered that the satellite communication module can be connected to the rescue processing device. When the satellite communication network cannot successfully reside in the current area, or when the satellite communication network can successfully reside in the current area, but the signal strength measurement result is less than a preset threshold, it is considered that the satellite communication module cannot be connected to the rescue processing device.

When the satellite communication module of the mobile phone can be connected to the rescue processing device, the first distress information can be sent to the rescue processing device by using the satellite communication module of the mobile phone. The first distress information can be sent in a form of a data service, can be sent in a form of a call, or can be sent in a form of a short message. Therefore, the set preset threshold can meet a network quality requirement of the data service, the call, or the short message.

1104A: After determining that the satellite communication module can be connected to the rescue processing device, the processing module of the mobile phone sends a first request to the satellite communication module.

The first request is used for requesting to send the first distress information to the rescue processing device. The first request includes the first distress information.

1104B: The satellite communication module of the mobile phone sends the first distress information to the rescue processing device in response to the first request.

1105A: The rescue processing device generates rescue information based on the first distress information, and sends the rescue information to the satellite communication module of the mobile phone.

1105B: After receiving the rescue information, the satellite communication module of the mobile phone sends the rescue information to the processing module of the mobile phone.

Usually, after the rescue processing device receives the first distress information sent by using the satellite communication module, the rescue processing device contacts a rescue team closest to the distressed person to provide rescue for the distressed person based on a sending location of the first distress information. After the rescue processing device determines a corresponding rescue plan and rescue team, the rescue processing device sends rescue information to the mobile phone.

Specifically, the rescue processing device feeds back the rescue information to the distressed person based on communication means of the received first distress information. Therefore, the satellite communication module of the mobile phone can receive the rescue information sent from the rescue processing device, and then the satellite communication module sends the rescue information to the processing module. The processing module displays the received rescue information to the user through a display of the mobile phone to inform the user of a current rescue progress.

Before the rescue team arrives, when a user status is consistent with a distress status in the first distress information, the following 1106 is performed.

1106: Based on the rescue information, the processing module of the mobile phone establishes a connection with the rescue team at specified time and through specified communication means.

After receiving the rescue information, the processing module analyzes the rescue information, and then establishes the rescue link with the rescue team through the communication means indicated by the rescue information based on the time point indicated in the rescue information. The rescue processing device usually indicates communication means between the rescue team and the distressed person based on the communication means of the received first distress information.

Therefore, the processing module can establish the rescue link with the rescue team by using the satellite communication module. When the satellite communication module indicated by the rescue information is not available, the processing module may attempt to establish a connection with the rescue team by using the module for communication between remote devices.

When the rescue link is successfully established, the distressed person can wait for rescue of the rescue team.

When the rescue link fails to be established, the processing module sends the first distress information to the rescue processing device again until the rescue link is successfully established. The re-sent first distress information may be sent by using any one of the short-range connection module, the cellular communication module, the satellite communication module, or the module for communication between remote devices.

Before the rescue team arrives, when the user status is inconsistent with the distress status in the first distress information, the following 1107 to 1110 are performed.

1107: The wearable smart device detects that a health status of the user changes, collects user health change data, generates second pre-distress information based on the user health change data, and sends the second pre-distress information to the processing module of the mobile phone by using the short-range connection module.

Refer to 708 for details.

1108: After receiving the second pre-distress information, the processing module of the mobile phone generates second distress information based on the second pre-distress information, and sends the second distress information to the rescue processing device.

For the part in which the processing module generates the second distress information based on the second pre-distress information, refer to a manner of generating the first distress information from the first pre-distress information, and see 703 for details.

After generating the second distress information, the processing module can sequentially try to send the second distress information to the rescue processing device by using the short-range connection module, the cellular communication module, the satellite communication module, and the module for communication between remote devices. After the second distress information is sent, 1105A to 1110 can be continued.

1109: The wearable smart device detects that a location of the user changes, generates third pre-distress information based on a changed user location state, and sends the third pre-distress information to the processing module of the mobile phone by using the short-range connection module.

Refer to 711 for details.

1110: After receiving the third pre-distress information, the processing module of the mobile phone generates third distress information based on the third pre-distress information, and sends the third distress information to the rescue processing device.

For the part in which the processing module generates the third distress information based on the third pre-distress information, refer to a manner of generating the first distress information from the first pre-distress information, and see 703 for details.

After generating the third distress information, the processing module can sequentially try to send the second distress information to the rescue processing device by using the short-range connection module, the cellular communication module, the satellite communication module, and the module for communication between remote devices. After the third distress information is sent, 1105A to 1110 can be continued.

It should be noted that, embodiments of this disclosure may not limit an execution sequence between 1107 and 1108 and 1109 and 1110. For example, 1107 and 1108 may be performed first, followed by 1109 and 1110. Alternatively, 1109 and 1110 may be performed first, followed by 1107 and 1108. Alternatively, 1107 and 1108 and 1109 and 1110 may be performed at the same time. Alternatively, only 1107 and 1108 are performed first, and 1109 and 1110 are not performed. Alternatively, only 1109 and 1110 are performed, and 1107 and 1108 are not performed. This may be specifically determined based on an actual use requirement.

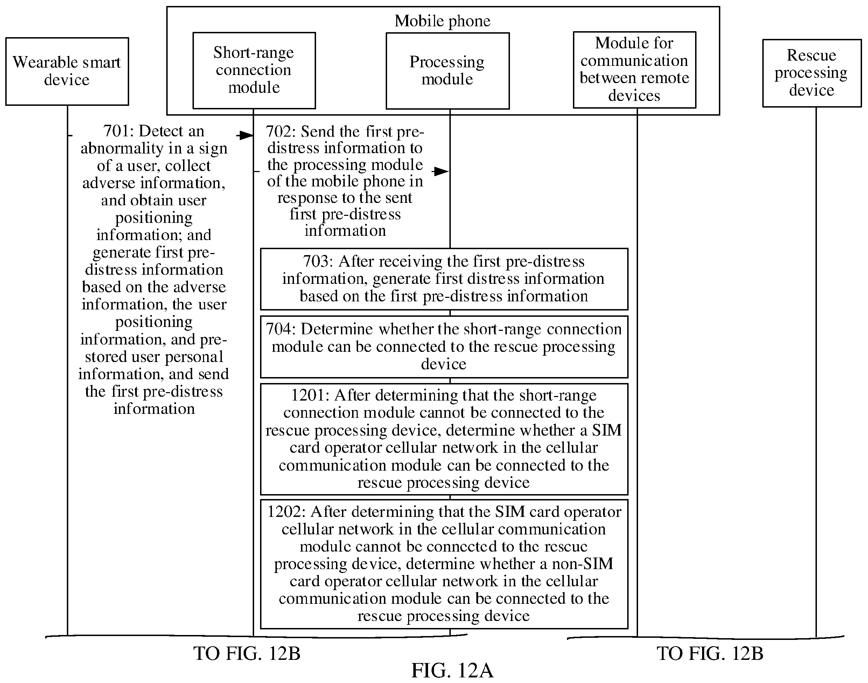

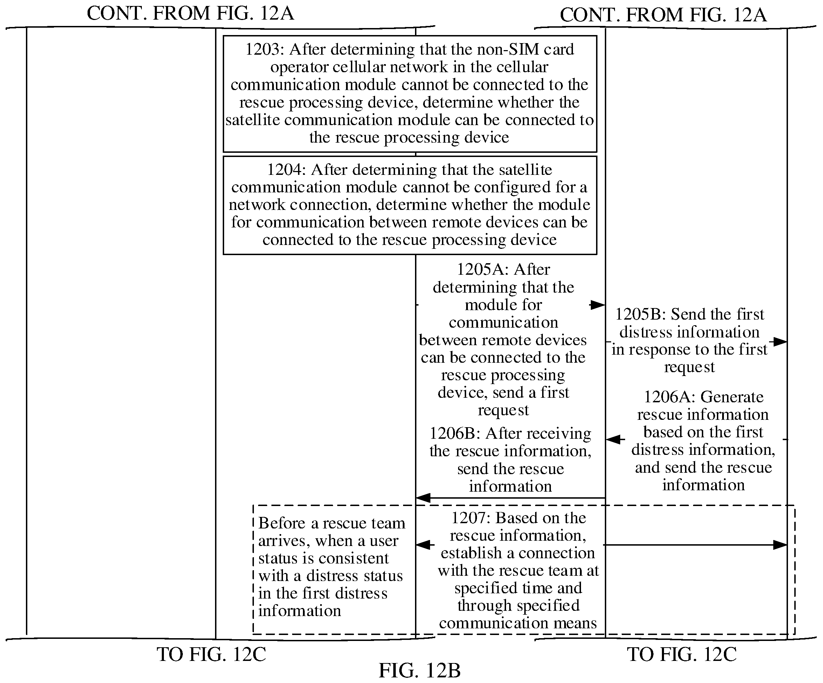

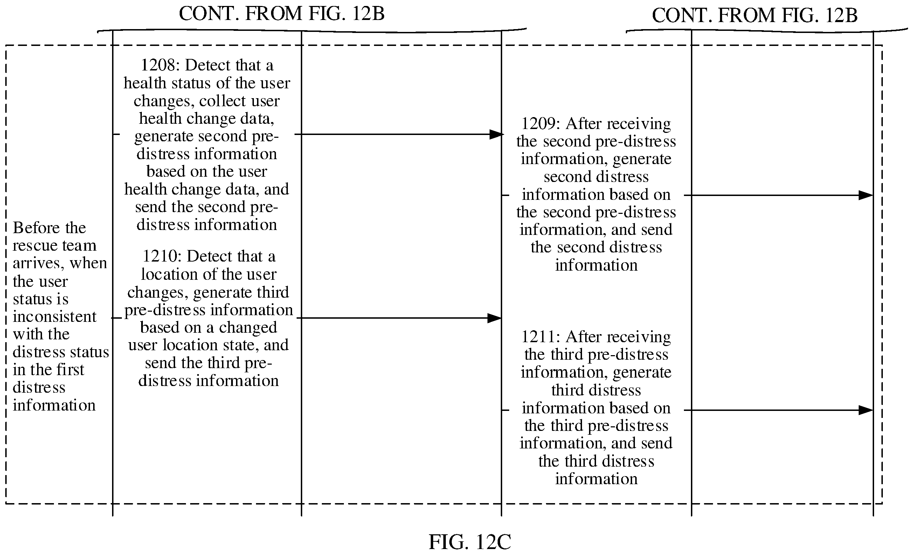

For example, in FIG. 12A to FIG. 12C, another distress information sending method provided by an embodiment of this application is described below. After 701 to 704, the method further includes:
1201: After determining that the short-range connection module cannot be connected to the rescue processing device, the processing module of the mobile phone determines whether a SIM card operator cellular network in the cellular communication module can be connected to the rescue processing device.
   Specifically, when the SIM card operator cellular network in the cellular communication module can be connected to the rescue processing device, the processing module can directly send the first distress information to the rescue processing device by using the SIM card operator cellular network in the cellular communication module without continuing to perform 1202 to 1205A.
1202: After determining that the SIM card operator cellular network in the cellular communication module cannot be connected to the rescue processing device, the processing module of the mobile phone determines whether a non-SIM card operator cellular network in the cellular communication module can be connected to the rescue processing device.
   Specifically, when the non-SIM card operator cellular network in the cellular communication module can be connected to the rescue processing device, the processing module can directly send the first distress information to the rescue processing device by using the non-SIM card operator cellular network in the cellular communication module without continuing to perform 1203 to 1205A.

1203. After determining that the non-SIM card operator cellular network in the cellular communication module cannot be connected to the rescue processing device, the processing module of the mobile phone determines whether the satellite communication module can be connected to the rescue processing device.

Specifically, when the satellite communication module can be connected to the rescue processing device, the processing module can directly send the first distress information to the rescue processing device by using the satellite communication module without continuing to perform 1204 and 1205A.

1204: After determining that the satellite communication module cannot be connected to the rescue processing device, the processing module of the mobile phone determines whether the module for communication between remote devices can be connected to the rescue processing device.

The mobile phone performs signal scanning by using the module for communication between remote devices. When another nearby peer device supporting communication between remote devices is obtained through scanning, the module for communication between remote devices of the mobile phone establishes a connection with the peer device. After the connection is established, the module for communication between remote devices of the mobile phone sends an inquiry instruction to the peer device, the inquiry instruction includes an address of the rescue processing device, and the inquiry instruction is used for inquiring whether the peer device can be connected to the rescue processing device. After receiving the inquiry instruction, the peer device determines whether a communication module of the peer device can be connected to the rescue processing device, and after obtaining a determining result, the peer device sends an inquiry result to the module for communication between remote devices of the mobile phone. The inquiry result is used to inform the mobile phone whether the peer device can be connected to the rescue processing device. When the peer device can be connected to the rescue processing device, the module for communication between remote devices of the mobile phone can send the first distress information to the peer device, and the peer device forwards the first distress information to the rescue processing device.

The communication module of the peer device may include a short-range connection unit, a cellular network unit, a satellite communication unit, a unit for communication between remote devices, and the like. The peer device determines whether the communication module of the peer device can be connected to the rescue processing device, and can sequentially determine whether the short-range connection unit, the cellular network unit, the satellite communication unit, and the unit for communication between remote devices can be connected to the rescue processing device. For a specific determining manner, refer to 704, 901, 1002, 1103, and 1204.

1205A: After determining that the module for communication between remote devices can be connected to the rescue processing device, the processing module of the mobile phone sends a first request to the module for communication between remote devices.

The first request is used for requesting to send the first distress information to the rescue processing device, and the first request includes the first distress information.

1205B: The module for communication between remote devices of the mobile phone sends the first distress information to the rescue processing device in response to the first request.

Specifically, after receiving the first request, the module for communication between remote devices of the mobile phone sends a first distress message in the first request to the peer device, and after receiving the first distress message, the peer device forwards the first distress message to the rescue processing device.

1206A: The rescue processing device generates rescue information based on the first distress information, and sends the rescue information to the module for communication between remote devices of the mobile phone.

1206B: After receiving the rescue information, the module for communication between remote devices of the mobile phone sends the rescue information to the processing module of the mobile phone.

Usually, after the rescue processing device receives the first distress information sent by using the module for communication between remote devices, the rescue processing device contacts a rescue team closest to the distressed person to provide rescue for the distressed person based on a sending location of the first distress information. After the rescue processing device determines a corresponding rescue plan and rescue team, the rescue processing device sends rescue information to the mobile phone.

Specifically, the rescue processing device feeds back the rescue information to the distressed person based on communication means of the received first distress information. Therefore, the module for communication between remote devices of the mobile phone can receive the rescue information sent from the rescue processing device, and then the module for communication between remote devices sends the rescue information to the processing module. The processing module displays the received rescue information to the user through a display of the mobile phone to inform the user of a current rescue progress.

Before the rescue team arrives, when a user status is consistent with a distress status in the first distress information, the following 1207 is performed.

1207: Based on the rescue information, the processing module of the mobile phone establishes a connection with the rescue team at specified time and through specified communication means.

After receiving the rescue information, the processing module analyzes the rescue information, and then establishes the rescue link with the rescue team through the communication means indicated by the rescue information based on the time point indicated in the rescue information.

The rescue processing device usually indicates communication means between the rescue team and the distressed person based on the communication means of the received first distress information. Therefore, the processing module can establish the rescue link with the rescue team by using the module for communication between remote devices.

When the rescue link is successfully established, the distressed person can wait for rescue of the rescue team.

When the rescue link fails to be established, the processing module sends the first distress information to the rescue processing device again until the rescue link is successfully established. The re-sent first distress information may be sent by using any one of the short-range connection module, the cellular communication module, the satellite communication module, or the module for communication between remote devices.

Before the rescue team arrives, when the user status is inconsistent with the distress status in the first distress information, the following 1208 to 1211 are performed.

1208: The wearable smart device detects that a health status of the user changes, collects user health change data, generates second pre-distress information based on the user health change data, and sends the second pre-distress information to the processing module of the mobile phone by using the short-range connection module.

Refer to 708 for details.

1209: After receiving the second pre-distress information, the processing module of the mobile phone generates second distress information based on the second pre-distress information, and sends the second distress information to the rescue processing device.

For the part in which the processing module generates the second distress information based on the second pre-distress information, refer to a manner of generating the first distress information from the first pre-distress information, and see 703 for details.

After generating the second distress information, the processing module can sequentially try to send the second distress information to the rescue processing device by using the short-range connection module, the cellular communication module, the satellite communication module, and the module for communication between remote devices. After the second distress information is sent, 1206A to 1211 can be continued.

1210: The wearable smart device detects that a location of the user changes, generates third pre-distress information based on a changed user location state, and sends the third pre-distress information to the processing module of the mobile phone by using the short-range connection module.

Refer to 712 for details.

1211: After receiving the third pre-distress information, the processing module of the mobile phone generates third distress information based on the third pre-distress information, and sends the third distress information to the rescue processing device.

For the part in which the processing module generates the third distress information based on the third pre-distress information, refer to a manner of generating the first distress information from the first pre-distress information, and see 703 for details.

After generating the third distress information, the processing module can sequentially try to send the second distress information to the rescue processing device by using the short-range connection module, the cellular communication module, the satellite communication module, and the module for communication between remote devices. After the third distress information is sent, 1206A to 1211 can be continued.

It should be noted that, embodiments of this disclosure may not limit an execution sequence between 1208 and 1209 and 1210 and 1211. For example, 1208 and 1209 may be performed first, followed by 1210 and 1211. Alternatively, 1210 and 1211 may be performed first, followed by 1208 and 1209. Alternatively. 1208 and 1209 and 1210 and 1211 may be performed at the same time. Alternatively, only 1208 and 1209 are performed first, and 1210 and 1211 are not performed. Alternatively, only 1210 and 1211 are performed, and 1208 and 1209 are not performed. This may be specifically determined based on an actual use requirement.

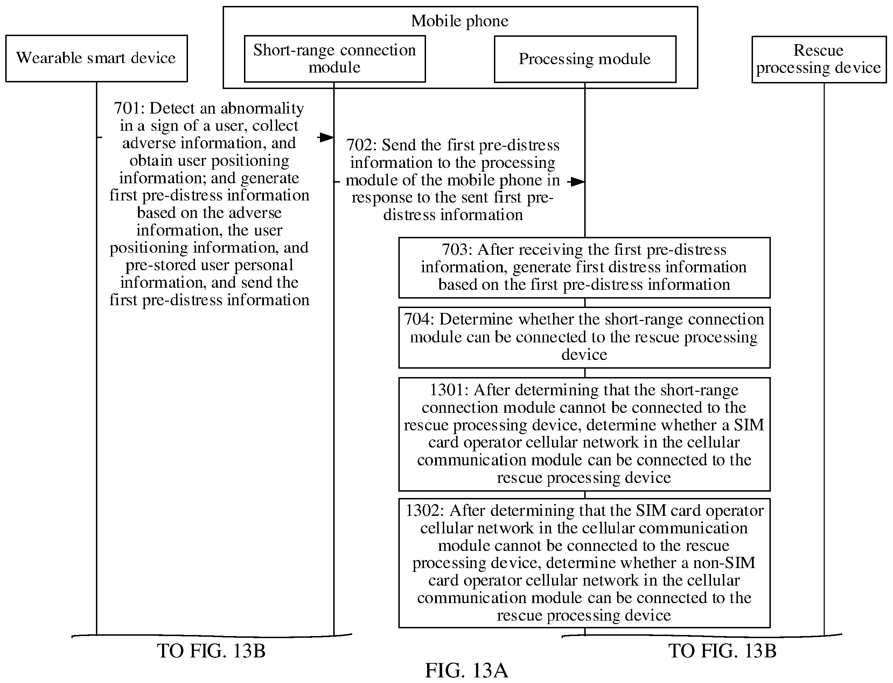

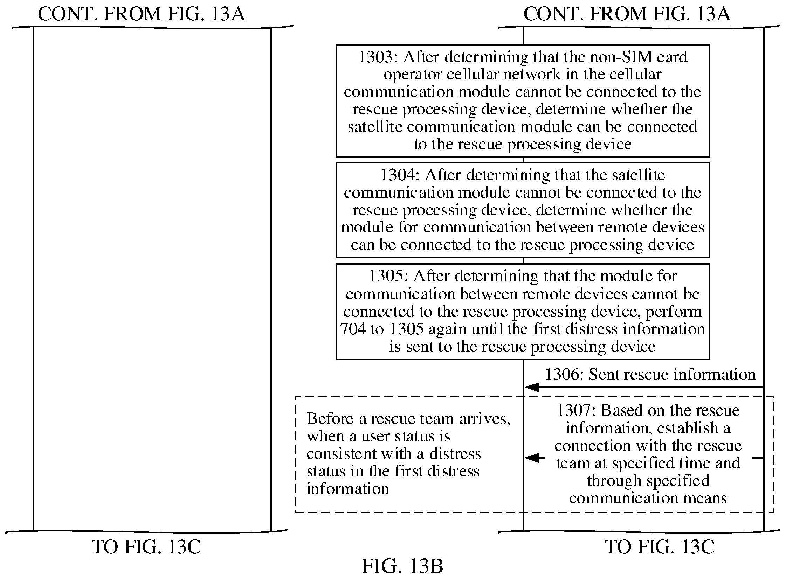

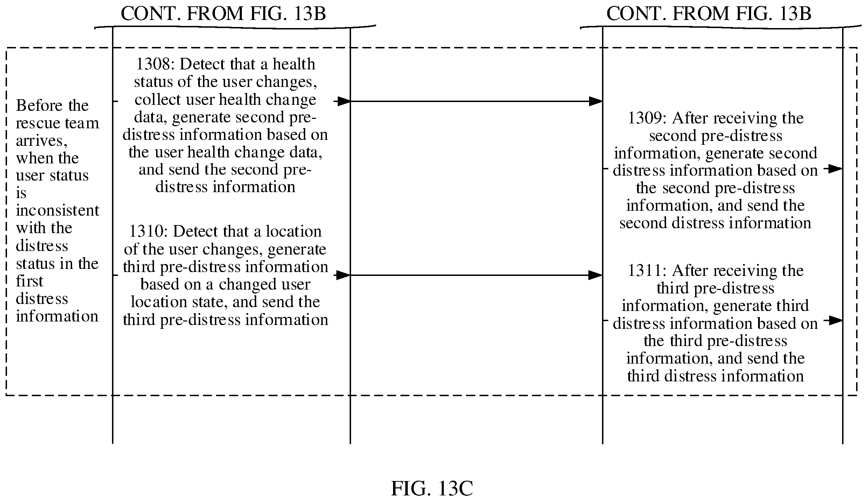

For example, in FIG. 13 A to FIG. 13C, another distress information sending method provided by an embodiment of this application is described below. After 701 to 704, the method further includes:
1301: After determining that the short-range connection module cannot be connected to the rescue processing device, the processing module of the mobile phone determines whether a SIM card operator cellular network in the cellular communication module can be connected to the rescue processing device.
   Specifically, when the SIM card operator cellular network in the cellular communication module can be connected to the rescue processing device, the processing module can directly send the first distress information to the rescue processing device by using the SIM card operator cellular network in the cellular communication module without continuing to perform 1302 to 1305.
1302: After determining that the SIM card operator cellular network in the cellular communication module cannot be connected to the rescue processing device, the processing module of the mobile phone determines whether a non-SIM card operator cellular network in the cellular communication module can be connected to the rescue processing device.
   Specifically, when the non-SIM card operator cellular network in the cellular communication module can be connected to the rescue processing device, the processing module can directly send the first distress information to the rescue processing device by using the non-SIM card operator cellular network in the cellular communication module without continuing to perform 1303 to 1305.
1303: After determining that the non-SIM card operator cellular network in the cellular communication module cannot be connected to the rescue processing device, the processing module of the mobile phone determines whether the satellite communication module can be connected to the rescue processing device.
   Specifically, when the satellite communication module can be connected to the rescue processing device, the processing module can directly send the first distress information to the rescue processing device by using the satellite communication module without continuing to perform 1304 and 1305.
1304: After determining that the satellite communication module cannot be connected to the rescue processing device, the processing module of the mobile phone determines whether the module for communication between remote devices can be connected to the rescue processing device.
   Specifically, when the module for communication between remote devices can be connected to the rescue processing device, the processing module can directly send the first distress information to the rescue processing device by using the module for communication between remote devices without further continuing to perform 1305.
1305: After determining that the module for communication between remote devices cannot be connected to the rescue processing device, the processing module of the mobile phone performs 704 to 1305 again until the first distress information is sent to the rescue processing device.

Specifically, the processing module starts the module for communication between remote devices to scan a surrounding device that also starts a module for communication between remote devices. At the same time, the processing module sets preset time T, and in the preset time T, when the device that starts the module for communication between remote devices is not obtained through scanning, it is considered that the module for communication between remote devices cannot be connected to the rescue processing device.

After determining that the module for communication between remote devices cannot be connected to the rescue processing device, the processing module continues to determine whether the short-range connection module, the cellular communication module, the satellite communication module, or the module for communication between remote devices can be connected to the rescue processing device according to 704 to 1305. When there is a module that is in the short-range connection module, the cellular communication module, the satellite communication module, or the module for communication between remote devices and that can be connected to the rescue processing device, the first distress information is sent to the rescue processing device by using the module, and the following 1306 to 1311 is continued. When there is no module that is in the short-range connection module, the cellular communication module, the satellite communication module, or the module for communication between remote devices and that can be connected to the rescue processing device, the processing module continues to execute 704 to 1305 until the first distress information is sent.

1306: Receive rescue information sent by the rescue processing device.

Usually, after the rescue processing device receives the first distress information sent by using any one of the short-range connection module, the cellular communication module, the satellite communication module, or the module for communication between remote devices, the rescue processing device contacts a rescue team closest to the distressed person to provide rescue for the distressed person based on a sending location of the first distress information. After the rescue processing device determines a corresponding rescue plan and rescue team, the rescue processing device sends rescue information to the mobile phone.

Specifically, the rescue processing device feeds back the rescue information to the distressed person based on communication means of the received first distress information. Then the module receiving the rescue information sends the rescue information to the processing module, and the processing module displays the received rescue information to the user through a display of the mobile phone to inform the user of a current rescue progress.

Before the rescue team arrives, when a user status is consistent with a distress status in the first distress information, the following 1307 is performed.

1307: Based on the rescue information, the processing module of the mobile phone establishes a connection with the rescue team at specified time and through specified communication means.

After receiving the rescue information, the processing module analyzes the rescue information, and then establishes the rescue link with the rescue team through the communication means indicated by the rescue information based on the time point indicated in the rescue information.

The rescue processing device usually indicates communication means between the rescue team and the distressed person based on the communication means of the received first distress information.

When the rescue link is successfully established, the distressed person can wait for rescue of the rescue team.

When the rescue link fails to be established, the processing module sends the first distress information to the rescue processing device again until the rescue link is successfully established. The re-sent first distress information may be sent by using any one of the short-range connection module, the cellular communication module, the satellite communication module, or the module for communication between remote devices.

Before the rescue team arrives, when the user status is inconsistent with the distress status in the first distress information, the following 1308 to 1311 are performed.

1308: The wearable smart device detects that a health status of the user changes, collects user health change data, generates second pre-distress information based on the user health change data, and sends the second pre-distress information to the processing module of the mobile phone by using the short-range connection module.

Refer to 708 for details.

1309: After receiving the second pre-distress information, the processing module of the mobile phone generates second distress information based on the second pre-distress information, and sends the second distress information to the rescue processing device.

For the part in which the processing module generates the second distress information based on the second pre-distress information, refer to a manner of generating the first distress information from the first pre-distress information, and see 703 for details.

After generating the second distress information, the processing module can sequentially try to send the second distress information to the rescue processing device by using the short-range connection module, the cellular communication module, the satellite communication module, and the module for communication between remote devices. After the second distress information is sent, 1306 to 1311 can be continued.

1310: The wearable smart device detects that a location of the user changes, generates third pre-distress information based on a changed user location state, and sends the third pre-distress information to the processing module of the mobile phone by using the short-range connection module.

Refer to 713 for details.

1311: After receiving the third pre-distress information, the processing module of the mobile phone generates third distress information based on the third pre-distress information, and sends the third distress information to the rescue processing device.

For the part in which the processing module generates the third distress information based on the third pre-distress information, refer to a manner of generating the first distress information from the first pre-distress information, and see 703 for details.

After generating the third distress information, the processing module can sequentially try to send the second distress information to the rescue processing device by using the short-range connection module, the cellular communication module, the satellite communication module, and the module for communication between remote devices. After the third distress information is sent, 1306 to 1311 can be continued.

It should be noted that, embodiments of this disclosure may not limit an execution sequence between 1308 and 1309 and 1310 and 1311. For example, 1308 and 1309 may be performed first, followed by 1310 and 1311. Alternatively, 1310 and 1311 may be performed first, followed by 1308 and 1309. Alternatively. 1308 and 1309 and 1310 and 1311 may be performed at the same time. Alternatively, only 1308 and 1309 are performed first, and 1310 and 1311 are not performed. Alternatively, only 1310 and 1311 are performed, and 1308 and 1309 are not performed. This may be specifically determined based on an actual use requirement.

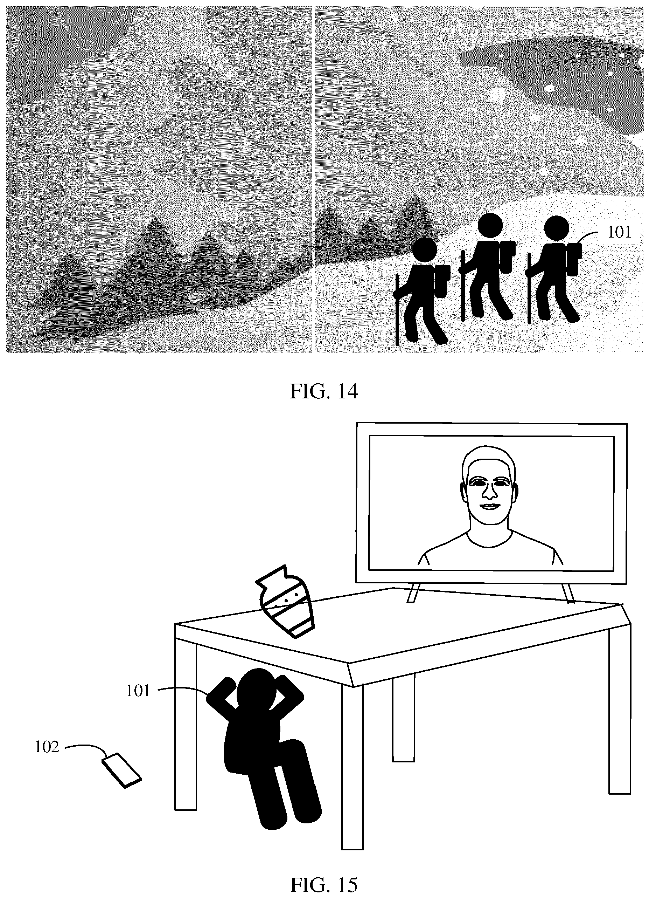

For example, FIG. 14 illustrates another possible distress scenario in which a user 101 and companions go hiking in the snow. In the process of hiking in the snow, the weather suddenly changed, and a blizzard began. Gradually, bodies of the user 101 and the companions could no longer support the user 101 and the companions to continue hiking. At this time, everyone stays in place to rest, and because a temperature in the snow is very low and there is no exercise, a body temperature of everyone begins to drop.

At this time, after a smart watch worn by the user 101 detects that a temperature drop of the user 101 exceeds a preset threshold, the smart watch automatically generates first pre-distress information including the temperature drop, and then sends the first pre-distress information to a mobile phone of the user 101, and then the mobile phone of the user 101 generates first distress information based on the first pre-distress information, and then the mobile phone traverses a short-range connection module, a cellular communication module, a satellite communication module, and a module for communication between remote devices to try to send the first distress information to a rescue processing device. After the first distress information is sent, the user 101 can obtain rescue information fed back by the rescue processing device, and then the user can wait for rescue in a manner indicated by the rescue information. Usually, the user 101 can only think of making a distress call to contact the outside world. This can make full use of a communication function of the mobile phone and make the user 101 and the companions be rescued in time.

For example, FIG. 15 illustrates another possible distress scenario in which a user 101 is watching television, suddenly feels a tremor, and then sees a vase on a table swaying.

At this time, the user 101 realized that an earthquake occurs, and quickly hides under the table. In the process of hiding, a mobile phone 102 of the user 101 falls out of a pocket. In the process of the earthquake, an accident occurs on the user 101, and a smart watch worn by the user 101 detects that a heartbeat frequency drop of the user 101 exceeds a preset threshold, and then the smart watch automatically generates first pre-distress information including the heartbeat frequency drop, and then sends the first pre-distress information to the mobile phone 102 of the user 101. At this time, although the user 101 cannot actively pick up the mobile phone 102 to call for help due to the accident, the mobile phone 102 can receive the first pre-distress information by adopting a solution provided by this application, so that the call for help can be completed without cooperation of the user. The mobile phone 102 then generates first distress information based on the first pre-distress information, and then the mobile phone traverses a short-range connection module, a cellular communication module, a satellite communication module, and a module for communication between remote devices in an attempt to send the first distress information to a rescue processing device. After the first distress information is sent, the user 101 can obtain rescue information fed back by the rescue processing device, and then the user 101 can wait for rescue in a manner indicated by the rescue information.

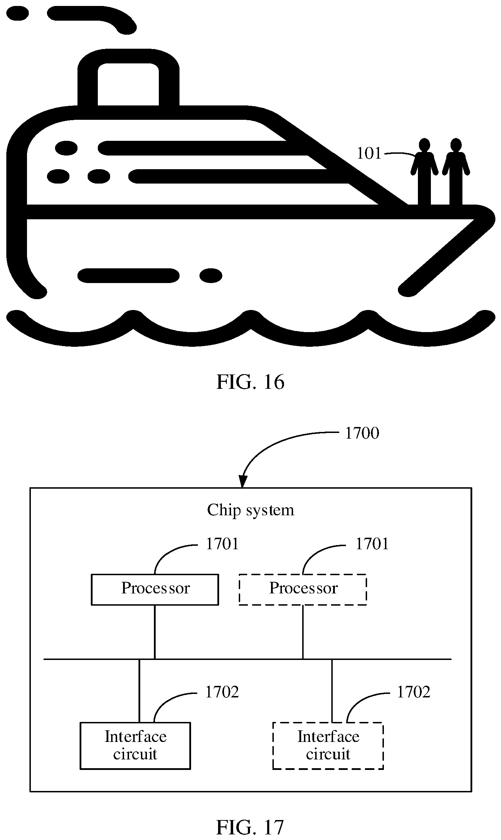

For example, FIG. 16 illustrates another possible distress scenario in which a user 101 and companions are on a ship on a long voyage and a device indicating a direction on the ship suddenly fails, resulting in that the captain is incapable of determining the direction of the ship. The user 101 then inputs custom information on a mobile phone, where the custom information is information about the current dilemma.

At this time, after a user interaction module of the mobile phone receives the custom information, the user interaction module sends the custom information to a processing module, and the processing module generates first distress information based on the custom information and a pre-stored personal profile. Then, the mobile phone traverses a short-range connection module, a cellular communication module, a satellite communication module, and a module for communication between remote devices in an attempt to send the first distress information to a rescue processing device. After the first distress information is sent, the user 101 can obtain rescue information fed back by the rescue processing device, and then the user can wait for rescue in a manner indicated by the rescue information.

That an embodiment of the application provides a distress information sending method may be specifically providing a switching mechanism of communication means. This switching mechanism of communication means is applied in a scenario of emergency rescue. By using the switching mechanism of communication means, all possible communication means of a terminal used by a distressed person can be traversed to send distress information. Therefore, missing an available communication network is avoided and the distressed person can be rescued in time.

It may be understood that to implement the foregoing functions, the second electronic device includes corresponding hardware structures and/or software modules for performing the functions. It should be readily appreciated by a person skilled in the art that the example units, algorithms, and steps described with reference to the embodiments disclosed in this specification can be implemented in the embodiments of this application by using hardware or a combination of hardware and computer software. Whether a function is performed by hardware or hardware driven by computer software depends on particular applications and design constraints of the technical solutions. A person skilled in the art may use different methods to implement the described functions for each particular application, but it should not be considered that the implementation goes beyond the scope of this application.

In embodiments of this application, the second electronic device may be divided into functional modules based on the foregoing method examples. For example, each functional module corresponding to each function may be obtained through division, or two or more functions may be integrated into one processing module. The integrated module may be implemented in the form of hardware, or may be implemented in a form of a software functional module. It should be noted that, in the embodiments of this application, the module division is an example, and is merely logical function division, and there may be other division manners during actual implementation.

Other embodiments of this application provide a second electronic device. The second electronic device may include: a communication module, a memory, and one or more processors. The communication module and the memory are coupled to the processor. The memory is configured to store computer program code, and the computer program code includes computer instructions.

Another embodiment of this application further provides a chip system. As shown in FIG. 17, the chip system includes at least one processor 1701 and at least one interface circuit 1702. The processor 1701 and the interface circuit 1702 may be interconnected by using a line. For example, the interface circuit 1702 may be configured to receive a signal from another apparatus. For another example, the interface circuit 1702 may be configured to send a signal to another apparatus (for example, the processor 1701).

For example, the interface circuit 1702 can read instructions stored in a memory of a device and send the instructions to the processor 1701. When the instructions are executed by the processor 1701, the second electronic device is enabled to perform the steps in the foregoing embodiments. Certainly, the chip system may further include another discrete component. This is not specifically limited in this embodiment of this application.

An embodiment of this application further provides a computer-readable storage medium, the computer-readable storage medium includes computer instructions, and when the computer instructions are run on a second electronic device, the second electronic device is enabled to perform the functions or steps performed by the second electronic device (for example, the mobile phone) in the method embodiments.

An embodiment of this application further provides a computer program product, and when the computer program product runs on a computer, the computer is enabled to perform the functions or steps performed by the second electronic device (for example, the mobile phone) in the method embodiments.

The foregoing descriptions about implementations allow a person skilled in the art to clearly understand that, for convenient and brief description, division of the foregoing function modules is taken as an example for illustration. In actual application, the foregoing functions can be allocated to different modules and implemented based on a requirement, that is, an inner structure of an apparatus is divided into different function modules to implement all or some of the functions described above.

In the several embodiments provided in this application, it should be understood that the disclosed apparatus and method may be implemented in other manners. For example, the described apparatus embodiments are merely examples. For example, division into the modules or units is merely logical function division and may be other division in actual implementation. For example, a plurality of units or components may be combined or integrated into another apparatus, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

The units described as separate parts may or may not be physically separate, and parts displayed as units may be one or more physical units, may be located in one place, or may be distributed on different places. Some or all of the units may be selected based on actual requirements to achieve the objectives of the solutions of embodiments.

In addition, functional units in embodiments of this application may be integrated into one processing unit, each of the units may exist alone physically, or two or more units are integrated into one unit. The integrated unit may be implemented in a form of hardware, or may be implemented in a form of a software functional unit.

When the integrated unit is implemented in the form of a software function unit and is sold or used as an independent product, the integrated unit may be stored in a readable storage medium. Based on such an understanding, the technical solutions of this application essentially, or the part contributing to the prior art, or all or some of the technical solutions may be implemented in the form of a software product. The software product is stored in a storage medium and includes several instructions for instructing a device (which may be a single-chip microcomputer, a chip or the like) or a processor (processor) to perform all or some of the steps of the methods described in embodiments of this application. The foregoing storage medium includes: any medium that can store program code, such as a USB flash drive, a removable hard disk, a read-only memory (read only memory, ROM), a random access memory (random access memory, RAM), a magnetic disk, or an optical disc.

The foregoing content is merely specific implementations of this application, but is not intended to limit the protection scope of embodiments of this application. Any variation or replacement within the technical scope disclosed in embodiments of this application shall fall within the protection scope of embodiments of this application. Therefore, the protection scope of this application shall be subject to the protection scope of the claims.

## Claims

1. A distress information sending method, wherein the method comprises:
obtaining (22), by a terminal device (22), first distress information, wherein the first distress information comprises at least one of adverse sign information or positioning information of a user (101);
selecting, by the terminal device (22), a first communication network from a plurality of communication networks according to a preset sequence, wherein the first communication network is any one of a short-range connection network, a cellular network, a satellite network, and a communication network between remote devices, and the communication network between remote devices is a point-to-point communication network established by the terminal device (22) and a rescue processing device (23) by using a preset frequency resource; and
when the terminal device (22) can be connected to the rescue processing device (23) by using the first communication network, sending, by the terminal device (22), the first distress information to the rescue processing device (23) by using the first communication network;
when the terminal device (22) cannot be connected to the rescue processing device (23) by using the first communication network, re-selecting, by the terminal device (22), a new first communication network from the plurality of communication networks according to the preset sequence,
the method **characterized by** the preset sequence from front to back being: the short-range connection network, the cellular network, the satellite network, and the communication network between remote devices.

2. The method according to claim 1, wherein before the obtaining, by a terminal device, first distress information, the method further comprises:
receiving (701), by the terminal device, first pre-distress information sent from a wearable smart device (21), wherein the first pre-distress information is generated based on the adverse sign information when the wearable smart device (21) detects an abnormality in a sign of the user; and
the obtaining, by a terminal device, first distress information comprises:
generating (703), by the terminal device, the first distress information based on the first pre-distress information.

3. The method according to claim 1 or 2, wherein selecting, by the terminal device, the first communication network from the plurality of communication networks according to the preset sequence; and when the terminal device can be connected to the rescue processing device by using the first communication network, sending, by the terminal device, the first distress information to the rescue processing device by using the first communication network; or when the terminal device cannot be connected to the rescue processing device by using the first communication network, the re-selecting, by the terminal device, a new first communication network from the plurality of communication networks according to the preset sequence comprises:
selecting, by the terminal device, the short-range connection network as the first communication network from the plurality of communication networks according to the preset sequence, and determining (704) whether the terminal device can be connected to the rescue processing device by using the short-range connection network;
when the terminal device can be connected to the rescue processing device by using the short-range connection network, sending (705B), by the terminal device, the first distress information to the rescue processing device by using the short-range connection network; or when the terminal device cannot be connected to the rescue processing device by using the short-range connection network, selecting the cellular network as the first communication network, and determining whether the terminal device can be connected to the rescue processing device by using the cellular network; and
when the terminal device can be connected to the rescue processing device by using the cellular network, sending (902B), by the terminal device, the first distress information to the rescue processing device by using the cellular network; or when the terminal device cannot be connected to the rescue processing device by using the cellular network, selecting the satellite network as the first communication network, and determining (1103) whether the terminal device can be connected to the rescue processing device by using the satellite network; and
when the terminal device can be connected to the rescue processing device by using the satellite network, sending (1104B), by the terminal device, the first distress information to the rescue processing device by using the satellite network; or when the terminal device cannot be connected to the rescue processing device by using the satellite network, selecting the communication network between remote devices as the first communication network, and determining (1204) whether the terminal device can be connected to the rescue processing device by using the communication network between remote devices; and
when the terminal device can be connected to the rescue processing device by using the communication network between remote devices, sending (1205B), by the terminal device, the first distress information to the rescue processing device by using the communication network between remote devices; or when the terminal device cannot be connected to the rescue processing device by using the communication network between remote devices, re-selecting (1305), by the terminal device, a new first communication network according to the preset sequence.

4. The method according to claim 3, wherein the determining whether the terminal device can be connected to the rescue processing device by using the communication network between remote devices comprises:
initiating, by the terminal device, scanning of a communication signal between the remote devices, and when the communication signal between the remote devices is obtained through scanning, establishing, by the terminal device, a connection with a peer device that sends the signal of the communication between the remote devices;
sending, by the terminal device, an inquiry instruction to the peer device, wherein the inquiry instruction is used for inquiring whether the peer device can be connected to the rescue processing device, and the inquiry instruction comprises an address of the rescue processing device; and
in response to an inquiry result sent by the peer device, when the peer device can be connected to the rescue processing device, determining that the terminal device can be connected to the rescue processing device by using the communication network between remote devices, when the peer device cannot be connected to the rescue processing device, determining that the terminal device cannot be connected to the rescue processing device by using the communication network between remote devices.

5. The method according to claim 3 or 4, wherein the determining whether the terminal device can be connected to the rescue processing device by using the cellular network comprises:
determining (901), by the terminal device, whether the terminal device can be connected to the rescue processing device by using a subscriber identity identification, SIM, card operator cellular network in the cellular network; and
when the terminal device can be connected to the rescue processing device by using the SIM card operator cellular network in the cellular network, determining that the first communication network is the SIM card operator cellular network in the cellular network; or when the terminal device cannot be connected to the rescue processing device by using the SIM card operator cellular network in the cellular network, determining (1002), by the terminal device, whether the terminal device can be connected to the rescue processing device by using a non-SIM card operator cellular network in the cellular network; and
when the terminal device can be connected to the rescue processing device by using a non-SIM card operator cellular network in the cellular network, determining that the first communication network is the non-SIM card operator cellular network in the cellular network; or
the method further comprises:
when the terminal device cannot be connected to the rescue processing device by using a non-SIM card operator cellular network in the cellular network, determining, by the terminal device, whether the terminal device can be connected to the rescue processing device by using the satellite network.

6. The method according to any one of claims 2 to 5, wherein the method further comprises:
receiving (708), by the terminal device, second pre-distress information sent from the wearable smart device, wherein the second pre-distress information is generated based on changed sign information when the wearable smart device detects that sign information of the user is inconsistent with the adverse sign information in the first pre-distress information; and
generating (709), by the terminal device, second distress information based on the second pre-distress information and sending the second distress information to the rescue processing device.

7. The method according to any one of claims 1 to 6, wherein the method further comprises:
receiving (710), by the terminal device, third pre-distress information sent from the wearable smart device, wherein the third pre-distress information is generated based on changed positioning information when the wearable smart device detects that positioning information of the user is inconsistent with the positioning information in the first pre-distress information; and
generating (711), by the terminal device, third distress information based on the third pre-distress information and sending the third distress information to the rescue processing device; or
when the terminal device detects that positioning information of the user is inconsistent with the positioning information in the first distress information, collecting the changed positioning information, generating fourth distress information based on the changed positioning information, and sending the fourth distress information to the rescue processing device.

8. The method according to any one of claims 1 to 7, wherein the method further comprises:
receiving (706A), by the terminal device, rescue information sent from the rescue processing device, wherein the rescue information is used to indicate the terminal device to establish contact with a rescue team.

9. The method according to claim 8, wherein the rescue information comprises rescue time and a communication mode between the rescue team and the user; and after the terminal device receives the rescue information sent from the rescue processing device, the method further comprises:
establishing (707), by the terminal device based on the communication mode between the rescue team and the user, a connection with the rescue team when the rescue time arrives.

10. The method according to claim 2, wherein the first pre-distress information further comprises at least one of user personal information, positioning information, emergency information, and situated environment information; the adverse sign information comprises at least one of a blood type, blood pressure, blood oxygen content, and a heart rate; the personal information comprises at least one of a gender and an age; the emergency information comprises at least one of a home address, an allergic reaction, an emergency contact name, and an emergency contact phone number; and the situated environment information comprises at least one of a photo of a situated environment and a video of the environment.

11. The method according to claim 6, wherein the sending the second distress information to the rescue processing device comprises:
determining, by the terminal device, a second communication network according to the preset sequence, wherein the second communication network is used for establishing a connection with the rescue processing device, and the second communication network is any one of the short-range connection network, the cellular network, the satellite network, and the communication network between remote devices.

12. A terminal device (22), wherein the terminal device (22) comprises: a wireless communication module (260), a memory (221), and one or more processors (210), and the wireless communication module (260) and the memory (221) are coupled to the processor (210), wherein
the memory (221) is configured to store computer program code, the computer program code comprises computer instructions, and when the computer instructions are executed by the processor (210), the terminal device (22) is enabled to perform the method according to any one of claims 1 to 11.

13. A distress information sending system, wherein the distress information sending system comprises a wearable smart device (21), a terminal device according to claim 12 (22), and a rescue processing device (23), and the wearable smart device (21), the terminal device (22), and the rescue processing device (23) are configured to perform the distress information sending method according to any one of claims 1 to 11.

## Patentansprüche

1. Verfahren zur Übermittlung von Notfallinformationen, wobei das Verfahren Folgendes umfasst:
Empfangen (22) von ersten Notfallinformationen durch ein Endgerät (22), wobei die ersten Notfallinformationen mindestens eines der folgenden enthalten: Informationen über kritische Vitalzeichen oder Positionsinformationen eines Nutzers (101);
Auswahl eines ersten Kommunikationsnetzwerks durch das Endgerät (22) aus einer Vielzahl von Kommunikationsnetzwerken gemäß einer voreingestellten Reihenfolge, wobei das erste Kommunikationsnetzwerk eines der folgenden ist: ein Nahbereichsverbindungsnetzwerk, ein Mobilfunknetz, ein Satellitennetzwerk oder ein Kommunikationsnetzwerk zwischen entfernten Geräten, wobei das Kommunikationsnetzwerk zwischen entfernten Geräten ein Punktzu-Punkt-Kommunikationsnetzwerk ist, das vom Endgerät (22) und einem Rettungsbearbeitungsgerät (23) mithilfe einer voreingestellten Frequenzressource aufgebaut wird; und
wenn das Endgerät (22) mithilfe des ersten Kommunikationsnetzwerks mit dem Rettungsbearbeitungsgerät (23) verbunden werden kann, Übermittlung der ersten Notfallinformationen durch das Endgerät (22) an das Rettungsbearbeitungsgerät (23) über das erste Kommunikationsnetzwerk;
wenn das Endgerät (22) mithilfe des ersten Kommunikationsnetzwerks nicht mit dem Rettungsbearbeitungsgerät (23) verbunden werden kann, erneute Auswahl eines neuen ersten Kommunikationsnetzwerks durch das Endgerät (22) aus der Vielzahl von Kommunikationsnetzwerken gemäß der voreingestellten Reihenfolge,
das Verfahren ist **dadurch gekennzeichnet, dass** die voreingestellte Reihenfolge von vorne nach hinten wie folgt lautet: das Nahbereichsverbindungsnetzwerk, das Mobilfunknetz, das Satellitennetzwerk und das Kommunikationsnetzwerk zwischen entfernten Geräten.

2. Das Verfahren gemäß Anspruch 1, wobei vor dem Empfang der ersten Notfallinformationen durch ein Endgerät das Verfahren weiter umfasst:
Empfangen (701) von ersten Vor-Notfallinformationen durch das Endgerät, die von einer tragbaren intelligenten Vorrichtung (21) gesendet wurden, wobei die ersten Vor-Notfallinformationen auf kritischen Vitalzeichendaten basieren, wenn die tragbare intelligente Vorrichtung (21) eine Anomalie an einem Zeichen des Nutzers erkennt; und
das Empfangen von ersten Notfallinformationen durch ein Endgerät umfasst:
Erzeugung (703) der ersten Notfallinformationen durch das Endgerät basierend auf den ersten Vor-Notfallinformationen.

3. Verfahren gemäß Anspruch 1 oder 2, wobei das Endgerät das erste Kommunikationsnetz aus der Vielzahl von Kommunikationsnetzen gemäß der voreingestellten Reihenfolge auswählt; und wenn das Endgerät über das erste Kommunikationsnetz mit der Rettungsverarbeitungseinrichtung verbunden werden kann, sendet das Endgerät die erste Notfallinformation über das erste Kommunikationsnetz an die Rettungsverarbeitungseinrichtung; oder wenn das Endgerät über das erste Kommunikationsnetz nicht mit der Rettungsverarbeitungseinrichtung verbunden werden kann, umfasst das erneute Auswählen eines neuen ersten Kommunikationsnetzes aus der Vielzahl von Kommunikationsnetzen durch das Endgerät gemäß der voreingestellten Reihenfolge:
Auswahl des Kurzstreckenverbindungsnetzes als erstes Kommunikationsnetz aus der Vielzahl von Kommunikationsnetzen gemäß der voreingestellten Reihenfolge durch das Endgerät und Feststellung (704), ob das Endgerät über das Kurzstreckenverbindungsnetz mit der Rettungsverarbeitungseinrichtung verbunden werden kann;
wenn das Endgerät über das Kurzstreckenverbindungsnetz mit der Rettungsverarbeitungseinrichtung verbunden werden kann, sendet das Endgerät die erste Notfallinformation über das Kurzstreckenverbindungsnetz an die Rettungsverarbeitungseinrichtung (705B); oder wenn das Endgerät über das Kurzstreckenverbindungsnetz nicht mit der Rettungsverarbeitungseinrichtung verbunden werden kann, Auswahl des Mobilfunknetzes als erstes Kommunikationsnetz und Feststellung, ob das Endgerät über das Mobilfunknetz mit der Rettungsverarbeitungseinrichtung verbunden werden kann; und
wenn das Endgerät über das Mobilfunknetz mit der Rettungsverarbeitungseinrichtung verbunden werden kann, sendet das Endgerät die erste Notfallinformation über das Mobilfunknetz an die Rettungsverarbeitungseinrichtung (902B); oder wenn das Endgerät über das Mobilfunknetz nicht mit der Rettungsverarbeitungseinrichtung verbunden werden kann, Auswahl des Satellitennetzes als erstes Kommunikationsnetz und Feststellung (1103), ob das Endgerät über das Satellitennetz mit der Rettungsverarbeitungseinrichtung verbunden werden kann; und
wenn das Endgerät über das Satellitennetz mit der Rettungsverarbeitungseinrichtung verbunden werden kann, sendet das Endgerät die erste Notfallinformation über das Satellitennetz an die Rettungsverarbeitungseinrichtung (1104B); oder wenn das Endgerät über das Satellitennetz nicht mit der Rettungsverarbeitungseinrichtung verbunden werden kann, Auswahl des Kommunikationsnetzes zwischen entfernten Geräten als erstes Kommunikationsnetz und Feststellung (1204), ob das Endgerät über das Kommunikationsnetz zwischen entfernten Geräten mit der Rettungsverarbeitungseinrichtung verbunden werden kann; und
wenn das Endgerät mit dem Rettungsverarbeitungsgerät über das Kommunikationsnetzwerk zwischen entfernten Geräten verbunden werden kann, wird durch das Endgerät die erste Notsignalinformation über das Kommunikationsnetzwerk zwischen entfernten Geräten an das Rettungsverarbeitungsgerät gesendet (1205B); oder wenn das Endgerät nicht mit dem Rettungsverarbeitungsgerät über das Kommunikationsnetzwerk zwischen entfernten Geräten verbunden werden kann, wählt das Endgerät nach der voreingestellten Reihenfolge ein neues erstes Kommunikationsnetzwerk erneut aus (1305).

4. Verfahren nach Anspruch 3, wobei das Bestimmen, ob das Endgerät mit dem Rettungsverarbeitungsgerät über das Kommunikationsnetzwerk zwischen entfernten Geräten verbunden werden kann, Folgendes umfasst:
Initiieren, durch das Endgerät, des Scannens eines Kommunikationssignals zwischen den entfernten Geräten; und wenn das Kommunikationssignal durch das Scannen gefunden wird, Aufbau einer Verbindung durch das Endgerät zu einem Peer-Gerät, das das Signal der Kommunikation zwischen den entfernten Geräten sendet;
Senden einer Abfrageanweisung durch das Endgerät an das Peer-Gerät, wobei die Abfrageanweisung dazu verwendet wird zu erfragen, ob das Peer-Gerät mit dem Rettungsverarbeitungsgerät verbunden werden kann, und die Abfrageanweisung die Adresse des Rettungsverarbeitungsgeräts enthält; und
Als Antwort auf ein Abfrageergebnis, das vom Peer-Gerät gesendet wird, wird, wenn das Peer-Gerät mit dem Rettungsverarbeitungsgerät verbunden werden kann, bestimmt, dass das Endgerät über das Kommunikationsnetzwerk zwischen entfernten Geräten mit dem Rettungsverarbeitungsgerät verbunden werden kann; wenn das Peer-Gerät nicht mit dem Rettungsverarbeitungsgerät verbunden werden kann, wird bestimmt, dass das Endgerät nicht über das Kommunikationsnetzwerk zwischen entfernten Geräten mit dem Rettungsverarbeitungsgerät verbunden werden kann.

5. Verfahren nach Anspruch 3 oder 4, wobei das Bestimmen, ob das Endgerät über das Mobilfunknetz mit dem Rettungsverarbeitungsgerät verbunden werden kann, Folgendes umfasst:
Bestimmen (901) durch das Endgerät, ob das Endgerät über ein Mobilfunknetz des SIM-Kartenbetreibers in dem Mobilfunknetz unter Verwendung der Teilnehmeridentifikation (SIM) mit dem Rettungsverarbeitungsgerät verbunden werden kann; und
Wenn das Endgerät über das Mobilfunknetz des SIM-Karten-Betreibers im Mobilfunknetz mit der Rettungsverarbeitungseinrichtung verbunden werden kann, wird festgestellt, dass das erste Kommunikationsnetz das Mobilfunknetz des SIM-Karten-Betreibers im Mobilfunknetz ist; oder wenn das Endgerät nicht über das Mobilfunknetz des SIM-Karten-Betreibers im Mobilfunknetz mit der Rettungsverarbeitungseinrichtung verbunden werden kann, wird (1002) durch das Endgerät festgestellt, ob das Endgerät über ein Mobilfunknetz eines Nicht-SIM-Karten-Betreibers im Mobilfunknetz mit der Rettungsverarbeitungseinrichtung verbunden werden kann; und
Wenn das Endgerät über ein Mobilfunknetz eines Nicht-SIM-Karten-Betreibers im Mobilfunknetz mit der Rettungsverarbeitungseinrichtung verbunden werden kann, wird festgestellt, dass das erste Kommunikationsnetz das Mobilfunknetz eines Nicht-SIM-Karten-Betreibers im Mobilfunknetz ist; oder
Das Verfahren umfasst ferner:
Wenn das Endgerät nicht über ein Mobilfunknetz eines Nicht-SIM-Karten-Betreibers im Mobilfunknetz mit der Rettungsverarbeitungseinrichtung verbunden werden kann, wird durch das Endgerät festgestellt, ob das Endgerät über das Satellitennetzwerk mit der Rettungsverarbeitungseinrichtung verbunden werden kann.

6. Verfahren nach einem der Ansprüche 2 bis 5, wobei das Verfahren ferner umfasst:
Empfangen (708) durch das Endgerät von zweiten Vor-Notfall-Informationen, die von der tragbaren Smartvorrichtung gesendet werden, wobei die zweiten Vor-Notfall-Informationen auf geänderten Vitalzeicheninformationen basieren, wenn die tragbare Smartvorrichtung erkennt, dass die Vitalzeicheninformationen des Benutzers nicht mit den negativen Vitalzeicheninformationen in den ersten Vor-Notfall-Informationen übereinstimmen; und
Erzeugen (709) durch das Endgerät von zweiten Notfallinformationen auf Basis der zweiten Vor-Notfall-Informationen und Senden der zweiten Notfallinformationen an die Rettungsverarbeitungseinrichtung.

7. Verfahren nach einem der Ansprüche 1 bis 6, wobei das Verfahren ferner umfasst:
Empfangen (710) durch das Endgerät von dritten Vor-Notfall-Informationen, die von der tragbaren Smartvorrichtung gesendet werden, wobei die dritten Vor-Notfall-Informationen auf geänderten Positionierungsinformationen basieren, wenn die tragbare Smartvorrichtung erkennt, dass die Positionierungsinformationen des Benutzers nicht mit den Positionierungsinformationen in den ersten Vor-Notfall-Informationen übereinstimmen; und
Erzeugen (711) durch das Endgerät von dritten Notfallinformationen auf Basis der dritten Vor-Notfall-Informationen und Senden der dritten Notfallinformationen an die Rettungsverarbeitungseinrichtung; oder
Wenn das Endgerät erkennt, dass die Positionsinformationen des Benutzers nicht mit den Positionsinformationen aus den ersten Notfalldaten übereinstimmen, werden die geänderten Positionsinformationen erfasst, vierte Notfalldaten basierend auf den geänderten Positionsinformationen erzeugt und die vierten Notfalldaten an die Rettungsbearbeitungseinheit gesendet.

8. Verfahren nach einem der Ansprüche 1 bis 7, wobei das Verfahren ferner umfasst:
Empfangen (706A) von Rettungsinformationen durch das Endgerät, wobei die Rettungsinformationen von der Rettungsbearbeitungseinheit gesendet werden und dazu dienen, das Endgerät anzuweisen, Kontakt mit einem Rettungsteam herzustellen.

9. Verfahren nach Anspruch 8, wobei die Rettungsinformationen die Rettungszeit und einen Kommunikationsmodus zwischen dem Rettungsteam und dem Benutzer umfassen; und nachdem das Endgerät die von der Rettungsbearbeitungseinheit gesendeten Rettungsinformationen empfängt, umfasst das Verfahren ferner:
Herstellen (707) einer Verbindung mit dem Rettungsteam durch das Endgerät auf Grundlage des Kommunikationsmodus zwischen dem Rettungsteam und dem Benutzer, wenn die Rettungszeit erreicht ist.

10. Verfahren nach Anspruch 2, wobei die ersten Vor-Notfalldaten mindestens eine der folgenden Informationen beinhalten: persönliche Angaben des Benutzers, Positionsinformationen, Notfallinformationen und Informationen zur Umgebung; die negativen Anzeichen umfassen mindestens einen der folgenden Werte: Blutgruppe, Blutdruck, Blutsauerstoffgehalt und Herzfrequenz; die persönlichen Angaben enthalten mindestens eines der folgenden Merkmale: Geschlecht und Alter; die Notfallinformationen umfassen mindestens eines der folgenden: Wohnanschrift, allergische Reaktion, Name des Notfallkontakts und Telefonnummer des Notfallkontakts; und die Umgebungsinformationen umfassen mindestens eines der folgenden: ein Foto der Umgebung und ein Video der Umgebung.

11. Verfahren nach Anspruch 6, wobei das Senden der zweiten Notfalldaten an die Rettungsbearbeitungseinheit umfasst:
Bestimmen eines zweiten Kommunikationsnetzes durch das Endgerät gemäß einer vorgegebenen Reihenfolge, wobei das zweite Kommunikationsnetz zur Verbindung mit der Rettungsbearbeitungseinheit genutzt wird und eines der folgenden ist:
Kurzstreckenverbindungsnetz, Mobilfunknetz, Satellitennetz und Kommunikationsnetz zwischen entfernten Geräten.

12. Endgerät (22), wobei das Endgerät (22) folgendes umfasst: ein drahtloses Kommunikationsmodul (260), einen Speicher (221) und einen oder mehrere Prozessoren (210), wobei das drahtlose Kommunikationsmodul (260) und der Speicher (221) an den Prozessor (210) gekoppelt sind, wobei
Der Speicher (221) ist so konfiguriert, dass er Computerprogrammcode speichert, wobei der Computerprogrammcode Computerbefehle umfasst und, wenn die Computerbefehle vom Prozessor (210) ausgeführt werden, das Endgerät (22) dazu befähigt wird, das Verfahren gemäß einem der Ansprüche 1 bis 11 auszuführen.

13. Ein Sendesystem für Notfallinformationen, wobei das Sendesystem für Notfallinformationen ein tragbares Smartgerät (21), ein Endgerät gemäß Anspruch 12 (22) und ein Rettungsverarbeitungsgerät (23) umfasst, und das tragbare Smartgerät (21), das Endgerät (22) und das Rettungsverarbeitungsgerät (23) dafür konfiguriert sind, das Verfahren zum Senden von Notfallinformationen gemäß einem der Ansprüche 1 bis 11 auszuführen.

## Revendications

1. Procédé d'envoi d'informations de détresse, dans lequel le procédé comprend :
obtention (22), par un dispositif terminal (22), de premières informations de détresse, lesdites premières informations de détresse comprenant au moins l'une des informations de signes anormaux ou des informations de positionnement d'un utilisateur (101) ;
sélection, par le dispositif terminal (22), d'un premier réseau de communication parmi une pluralité de réseaux de communication selon une séquence prédéfinie, ledit premier réseau de communication étant l'un quelconque d'un réseau de connexion à courte portée, d'un réseau cellulaire, d'un réseau satellite ou d'un réseau de communication entre dispositifs distants, et le réseau de communication entre dispositifs distants étant un réseau de communication point à point établi par le dispositif terminal (22) et un dispositif de traitement de sauvetage (23) en utilisant une ressource de fréquence prédéfinie ; et
lorsque le dispositif terminal (22) peut être connecté au dispositif de traitement de sauvetage (23) en utilisant le premier réseau de communication, envoi, par le dispositif terminal (22), des premières informations de détresse au dispositif de traitement de sauvetage (23) via le premier réseau de communication ;
lorsque le dispositif terminal (22) ne peut pas être connecté au dispositif de traitement de sauvetage (23) en utilisant le premier réseau de communication, nouvelle sélection, par le dispositif terminal (22), d'un nouveau premier réseau de communication parmi la pluralité de réseaux de communication selon la séquence prédéfinie,
le procédé, **caractérisé en ce que** la séquence prédéfinie de l'avant vers l'arrière est : le réseau de connexion à courte portée, le réseau cellulaire, le réseau satellite et le réseau de communication entre dispositifs distants.

2. Procédé selon la revendication 1, dans lequel, avant l'obtention, par un dispositif terminal, des premières informations de détresse, le procédé comprend en outre :
réception (701), par le dispositif terminal, de premières informations préalables de détresse envoyées par un dispositif intelligent portable (21), lesdites premières informations préalables de détresse étant générées sur la base des informations de signes anormaux lorsque le dispositif intelligent portable (21) détecte une anomalie dans un signe de l'utilisateur ; et
l'obtention, par un dispositif terminal, des premières informations de détresse comprend :
génération (703), par le dispositif terminal, des premières informations de détresse sur la base des premières informations préalables de détresse.

3. Le procédé selon la revendication 1 ou 2, dans lequel le dispositif terminal sélectionne le premier réseau de communication parmi la pluralité de réseaux de communication selon la séquence prédéfinie ; et lorsque le dispositif terminal peut être connecté à l'appareil de traitement de secours en utilisant le premier réseau de communication, le dispositif terminal envoie la première information de détresse à l'appareil de traitement de secours en utilisant le premier réseau de communication ; ou lorsque le dispositif terminal ne peut pas être connecté à l'appareil de traitement de secours en utilisant le premier réseau de communication, la nouvelle sélection par le dispositif terminal, d'un nouveau premier réseau de communication parmi la pluralité de réseaux de communication selon la séquence prédéfinie, comprend :
sélection, par le dispositif terminal, du réseau de connexion à courte portée comme premier réseau de communication parmi la pluralité de réseaux de communication selon la séquence prédéfinie, et détermination (704) de la capacité du dispositif terminal à se connecter à l'appareil de traitement de secours en utilisant le réseau de connexion à courte portée ;
lorsque le dispositif terminal peut être connecté à l'appareil de traitement de secours en utilisant le réseau de connexion à courte portée, envoi (705B), par le dispositif terminal, de la première information de détresse à l'appareil de traitement de secours en utilisant ce réseau ; ou lorsque le dispositif terminal ne peut pas être connecté à l'appareil de traitement de secours en utilisant le réseau de connexion à courte portée, sélection du réseau cellulaire comme premier réseau de communication, et détermination de la capacité du dispositif terminal à se connecter à l'appareil de traitement de secours en utilisant le réseau cellulaire ; et
lorsque le dispositif terminal peut être connecté à l'appareil de traitement de secours en utilisant le réseau cellulaire, envoi (902B), par le dispositif terminal, de la première information de détresse à l'appareil de traitement de secours en utilisant le réseau cellulaire ; ou lorsque le dispositif terminal ne peut pas être connecté à l'appareil de traitement de secours en utilisant le réseau cellulaire, sélection du réseau satellite comme premier réseau de communication, et détermination (1103) de la capacité du dispositif terminal à se connecter à l'appareil de traitement de secours en utilisant le réseau satellite ; et
lorsque le dispositif terminal peut être connecté à l'appareil de traitement de secours en utilisant le réseau satellite, envoi (1104B), par le dispositif terminal, de la première information de détresse à l'appareil de traitement de secours en utilisant le réseau satellite ; ou lorsque le dispositif terminal ne peut pas être connecté à l'appareil de traitement de secours en utilisant le réseau satellite, sélection du réseau de communication entre dispositifs distants comme premier réseau de communication, et détermination (1204) de la capacité du dispositif terminal à se connecter à l'appareil de traitement de secours en utilisant le réseau de communication entre dispositifs distants ; et
lorsque le dispositif terminal peut être connecté au dispositif de traitement de secours en utilisant le réseau de communication entre dispositifs distants, en envoyant (1205B), par le dispositif terminal, la première information de détresse au dispositif de traitement de secours via le réseau de communication entre dispositifs distants ; ou lorsque le dispositif terminal ne peut pas être connecté au dispositif de traitement de secours par le réseau de communication entre dispositifs distants, en ré-sélectionnant (1305), par le dispositif terminal, un nouveau premier réseau de communication selon la séquence prédéfinie.

4. Procédé selon la revendication 3, dans lequel la détermination de la possibilité pour le dispositif terminal d'être connecté au dispositif de traitement de secours par le réseau de communication entre dispositifs distants comprend :
le lancement, par le dispositif terminal, du balayage d'un signal de communication entre dispositifs distants, et lorsque le signal de communication entre dispositifs distants est obtenu par balayage, l'établissement, par le dispositif terminal, d'une connexion avec un dispositif pair qui envoie le signal de communication entre dispositifs distants ;
l'envoi, par le dispositif terminal, d'une instruction d'interrogation au dispositif pair, l'instruction d'interrogation servant à demander si le dispositif pair peut être connecté au dispositif de traitement de secours, et cette instruction d'interrogation comprenant une adresse du dispositif de traitement de secours ; et
en réponse à un résultat d'interrogation envoyé par le dispositif pair, lorsque le dispositif pair peut être connecté au dispositif de traitement de secours, il est déterminé que le dispositif terminal peut être connecté au dispositif de traitement de secours via le réseau de communication entre dispositifs distants, et lorsque le dispositif pair ne peut pas être connecté au dispositif de traitement de secours, il est déterminé que le dispositif terminal ne peut pas être connecté au dispositif de traitement de secours via le réseau de communication entre dispositifs distants.

5. Procédé selon la revendication 3 ou 4, dans lequel la détermination de la possibilité pour le dispositif terminal d'être connecté au dispositif de traitement de secours via le réseau cellulaire comprend :
la détermination (901), par le dispositif terminal, de la possibilité pour le dispositif terminal d'être connecté au dispositif de traitement de secours en utilisant un réseau cellulaire d'opérateur de carte SIM d'identification d'abonné dans le réseau cellulaire ; et
lorsque le dispositif terminal peut être connecté au dispositif de traitement de secours en utilisant le réseau cellulaire de l'opérateur de carte SIM dans le réseau cellulaire, déterminer que le premier réseau de communication est le réseau cellulaire de l'opérateur de carte SIM dans le réseau cellulaire ; ou lorsque le dispositif terminal ne peut pas être connecté au dispositif de traitement de secours en utilisant le réseau cellulaire de l'opérateur de carte SIM dans le réseau cellulaire, déterminer (1002), par le dispositif terminal, si le dispositif terminal peut être connecté au dispositif de traitement de secours en utilisant un réseau cellulaire d'un opérateur autre que celui de la carte SIM dans le réseau cellulaire ; et
lorsque le dispositif terminal peut être connecté au dispositif de traitement de secours en utilisant un réseau cellulaire d'un opérateur autre que celui de la carte SIM dans le réseau cellulaire, déterminer que le premier réseau de communication est le réseau cellulaire d'un opérateur autre que celui de la carte SIM dans le réseau cellulaire ; ou
le procédé comprend en outre :
lorsque le dispositif terminal ne peut pas être connecté au dispositif de traitement de secours en utilisant un réseau cellulaire d'un opérateur autre que celui de la carte SIM dans le réseau cellulaire, déterminer, par le dispositif terminal, si le dispositif terminal peut être connecté au dispositif de traitement de secours en utilisant le réseau satellite.

6. Procédé selon l'une quelconque des revendications 2 à 5, dans lequel le procédé comprend en outre :
recevoir (708), par le dispositif terminal, une deuxième information de pré-détresse envoyée par le dispositif intelligent portable, la deuxième information de pré-détresse étant générée sur la base d'informations de signes modifiées lorsque le dispositif intelligent portable détecte que les informations de signes de l'utilisateur ne sont pas conformes aux informations de signes indésirables de la première information de pré-détresse ; et
générer (709), par le dispositif terminal, une deuxième information de détresse sur la base de la deuxième information de pré-détresse et envoyer la deuxième information de détresse au dispositif de traitement de secours.

7. Procédé selon l'une quelconque des revendications 1 à 6, dans lequel le procédé comprend en outre :
recevoir (710), par le dispositif terminal, une troisième information de pré-détresse envoyée par le dispositif intelligent portable, la troisième information de pré-détresse étant générée sur la base d'informations de positionnement modifiées lorsque le dispositif intelligent portable détecte que les informations de positionnement de l'utilisateur ne correspondent pas aux informations de positionnement de la première information de pré-détresse ; et
générer (711), par le dispositif terminal, une troisième information de détresse sur la base de la troisième information de pré-détresse et envoyer la troisième information de détresse au dispositif de traitement de secours ; ou
lorsque le dispositif terminal détecte que les informations de localisation de l'utilisateur sont incohérentes avec les informations de localisation présentes dans la première information de détresse, il collecte les informations de localisation modifiées, génère une quatrième information de détresse sur la base des informations de localisation modifiées, et envoie la quatrième information de détresse au dispositif de traitement de secours.

8. Procédé selon l'une quelconque des revendications 1 à 7, dans lequel le procédé comprend en outre :
réception (706A), par le dispositif terminal, des informations de secours envoyées par le dispositif de traitement de secours, les informations de secours étant utilisées pour indiquer au dispositif terminal d'établir le contact avec une équipe de secours.

9. Procédé selon la revendication 8, dans lequel les informations de secours comprennent une heure de secours et un mode de communication entre l'équipe de secours et l'utilisateur ; et après que le dispositif terminal reçoit les informations de secours envoyées par le dispositif de traitement de secours, le procédé comprend en outre :
établissement (707), par le dispositif terminal, sur la base du mode de communication entre l'équipe de secours et l'utilisateur, d'une connexion avec l'équipe de secours lorsque l'heure de secours est arrivée.

10. Procédé selon la revendication 2, dans lequel la première information pré-détresse comprend en outre au moins une des informations suivantes : informations personnelles de l'utilisateur, informations de localisation, informations d'urgence et informations sur l'environnement situé ; les informations sur les signes défavorables comprennent au moins l'un des types sanguins, la pression artérielle, le contenu en oxygène du sang et la fréquence cardiaque ; les informations personnelles comprennent au moins le sexe et l'âge ; les informations d'urgence comprennent au moins une adresse personnelle, une réaction allergique, un nom de contact d'urgence et un numéro de téléphone d'urgence ; et les informations sur l'environnement situé comprennent au moins une photo de l'environnement situé et une vidéo de l'environnement.

11. Procédé selon la revendication 6, dans lequel l'envoi de la deuxième information de détresse au dispositif de traitement de secours comprend :
détermination, par le dispositif terminal, d'un second réseau de communication selon la séquence prédéfinie, le second réseau de communication étant utilisé pour établir une connexion avec le dispositif de traitement de secours et étant n'importe lequel du réseau de connexion à courte portée, du réseau cellulaire, du réseau satellite et du réseau de communication entre dispositifs distants.

12. Un dispositif terminal (22), dans lequel le dispositif terminal (22) comprend : un module de communication sans fil (260), une mémoire (221) et un ou plusieurs processeurs (210), et le module de communication sans fil (260) et la mémoire (221) sont couplés au(x) processeur(s) (210), dans lequel
la mémoire (221) est configurée pour stocker un code de programme informatique, le code de programme informatique comprend des instructions informatiques, et lorsque les instructions informatiques sont exécutées par le processeur (210), le dispositif terminal (22) est capable d'exécuter la méthode selon l'une quelconque des revendications 1 à 11.

13. Un système d' envoi d' informations de détresse, dans lequel le système d'envoi d' informations de détresse comprend un dispositif intelligent portable (21), un dispositif terminal selon la revendication 12 (22), et un dispositif de traitement de secours (23), et le dispositif intelligent portable (21), le dispositif terminal (22), et le dispositif de traitement de secours (23) sont configurés pour exécuter la méthode d' envoi d' informations de détresse selon l'une quelconque des revendications 1 à 11.
